(19) Europäisches Patentamt
European Patent Office
Office européen des brevets

(11) EP 1 674 074 A1

(12) DEMANDE DE BREVET EUROPEEN

(43) Date de publication:
28.06.2006 Bulletin 2006/26

(51) Int Cl.:
*A61K 8/60* (2006.01)

(21) Numéro de dépôt: 05292741.5

(22) Date de dépôt: 20.12.2005

(84) Etats contractants désignés:
AT BE BG CH CY CZ DE DK EE ES FI FR GB GR HU IE IS IT LI LT LU LV MC NL PL PT RO SE SI SK TR
Etats d'extension désignés:
AL BA HR MK YU

(30) Priorité: 23.12.2004 FR 0453189

(71) Demandeur: L'ORÉAL
75008 Paris (FR)

(72) Inventeurs:
- Lalleman, Boris
75015 Paris (FR)
- Hercouet, Leila
93360 Neuilly Plaisance (FR)

(74) Mandataire: Fevrier, Murielle Françoise E.
L'OREAL - D.I.P.I.
25-29 Quai Aulagnier
92600 Asnières (FR)

# (54) Traitement de fibres kératiniques colorées avec une composition comprenant un tensioactif non ionique particuler et/ou amphotère et/ou anionique doux particulier et utilisation pour protéger la couleur

(57) La présente invention a pour objet un procédé de traitement de fibres kératiniques humaines dans lequel on met en oeuvre les étapes suivantes :

a) on réalise une étape de coloration desdites fibres au moyen d'une composition prête à l'emploi à base d'une composition colorante comportant au moins une base d'oxydation du type paraphénylènediamine substituée par au moins un radical cationique ;
b) éventuellement, on lave les fibres avec une composition lavante (A) comprenant au moins un tensioactif détergent anionique choisi parmi les alkyléthers sulfate et/ou les alkyl sulfate, on les rince et éventuellement on les sèche ou on les laisse sécher ;
c) on lave les fibres avec une composition lavante (B) comprenant au moins un tensioactif amphotère et/ou au moins un tensioactif zwittérionique et/ou au moins un tensioactif anionique doux et/ou au moins un tensioactif non ionique choisi parmi les alkylpolyglucosides, les tensioactifs monoglycérolés ou polyglycérolés, puis on rince les fibres et on les sèche ou on les laisse sécher.

Elle a de même pour objet un kit pour la mise en oeuvre de ce procédé.

Enfin, elle concerne l'utilisation d'une composition lavante comprenant au moins un tensioactif amphotère, zwittérionique, anionique doux et/ou non ionique particulier, pour la protection de la coloration de fibres kératiniques, obtenue au moyen d'une composition de coloration comprenant au moins une base d'oxydation du type paraphénylènediamine substituée par au moins un radical cationique, ainsi que pour la limitation du dégorgement.

EP 1 674 074 A1

## Description

La présente invention a pour objet un procédé de traitement de fibres kératiniques colorées ainsi que l'utilisation de tensioactifs amphotères et/ou zwittérioniques et/ou anioniques doux et/ou non ioniques particuliers, ou leurs mélanges, pour protéger la coloration desdites fibres.

Plus précisément, le domaine de l'invention est celui du traitement des fibres kératiniques colorées, de préférence de fibres kératiniques humaines, comme notamment les cheveux.

Il est connu de teindre les fibres kératiniques et en particulier les cheveux humains avec des compositions tinctoriales contenant des précurseurs de colorant d'oxydation, appelés généralement bases d'oxydation, tels que des ortho ou para-phénylènediamines, des ortho ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-diphénols et certains composés hétérocycliques tels que des composés indoliques.

La coloration obtenue à partir de ce type de composition est une coloration permanente. Cependant, elle a tendance à s'affadir au cours du temps, en particulier eu fur et à mesure des shampooings.

Des shampooings peu agressifs ont été développés pour remédier à ce problème de la dégradation de la coloration lors des shampooings. Par exemple, la demande de brevet WO 2001/097759 décrit des shampoings non agressifs à base de tensio-actifs dérivés de sucre choisis parmi les alkyl et alcényl oligoglucoside ou un tensio-actif N-alkylpolyhydroxyalkylamide d'acide gras, ces tensioactifs étant associés à un glycéride partiel d'acide gras.

Selon ce document, ce shampooing permet d'améliorer la tenue aux lavages des couleurs et d'augmenter l'intensité de la coloration quel que soit le type de coloration mise en oeuvre.

Malgré le développement de ce type de shampooing, le phénomène de dégradation de la couleur lors des shampooings est toujours présent, en particulier sur les cheveux sensibilisés.

Le but de la présente invention est de développer de nouvelles techniques afin de remédier au problème lié à la dégradation de la couleur, shampooing après shampooing, tout en conservant de bonnes qualités d'usages du shampooing.

La présente invention a donc notamment pour objet de proposer un procédé de traitement des fibres kératiniques colorées permettant d'avoir un phénomène limité de dégorgement de la couleur lors des shampooings tout en bénéficiant de bonnes qualités l'usages pour lesdits shampooings.

Ainsi, la présente invention a pour objet un procédé de traitement de fibres kératiniques humaines dans lequel on met en oeuvre les étapes suivantes :

a) on réalise une étape de coloration desdites fibres au moyen d'une composition prête à l'emploi à base d'une composition colorante comportant au moins une base d'oxydation du type paraphénylène diamine substituée par au moins un radical cationique ;

b) éventuellement, on lave les fibres avec une composition lavante (A) comprenant au moins un tensioactif détergent anionique choisi parmi les alkyléthers sulfate et/ou les alkyl sulfate, on les rince et éventuellement on les sèche ou on les laisse sécher ;

c) on lave les fibres avec une composition lavante (B) comprenant au moins un tensioactif amphotère, et/ou au moins un tensioactif zwittérionique, et/ou au moins un tensioactif anionique doux, et/ou au moins un tensioactif non ionique choisi parmi les alkylpolyglucosides, les tensioactifs monoglycérolés ou polyglycérolés, puis on rince les fibres et on les sèche ou on les laisse sécher.

Elle a de même pour objet un kit pour la mise en oeuvre dudit procédé, comprenant une composition colorante et éventuellement une composition comprenant au moins un agent oxydant ; éventuellement au moins une composition lavante (A), et au moins une composition lavante (B).

Elle a enfin pour objet l'utilisation d'une composition lavante comprenant à titre de tensioactif, au moins un tensioactif amphotère et/ou au moins un tensioactif zwittérionique et/ou au moins un tensioactif anionique doux et/ou au moins un tensioactif non ionique exclusivement choisi parmi les alkylpolyglycosides, les tensioactifs monoglycérolés ou polyglycérolés, ou leurs mélanges, pour la limitation du dégorgement de la coloration et/ou la protection de la coloration des fibres kératiniques, en relation avec l'utilisation préalable d'une composition de coloration comprenant au moins une base d'oxydation du type paraphénylène diamine substituée par au moins un radical cationique.

On a en effet remarqué, de manière surprenante que l'emploi d'une composition lavante comprenant au moins un tensioactif non ionique particulier, et/ou au moins un tensioactif amphotère ou zwittérionique, et/ou au moins un tensioactif anionique doux, permettait de diminuer le phénomène de dégorgement de la couleur.

On a aussi constaté, par voie de conséquence, que le moindre dégorgement du ou des colorants se traduisait par un risque de tâchage de la peau et des tissus moins important.

Mais d'autres avantages et caractéristiques de l'invention apparaîtront plus clairement à la lecture de la description et des exemples qui vont suivre.

Ainsi que cela a été indiqué auparavant, le procédé selon l'invention consiste, dans une première étape, à réaliser une étape de coloration desdites fibres au moyen d'une composition prête à l'emploi à base d'une composition colorante comportant au moins une base d'oxydation du type paraphénylène diamine substituée par au moins un radical cationique.

Un tel procédé est particulièrement efficace sur des cheveux sensibilisés. On entend par cheveux sensibilisés des cheveux ayant par exemple subi des traitements cosmétiques modifiant chimiquement le cheveu tels qu'une permanente, une décoloration, un défrisage ou une combinaison de ces traitements ou par exemple l'action prolongée de la lumière ou l'action mécanique du brossage répété.

Les bases d'oxydation du type para-phénylènediamine substituées par un ou plusieurs radicaux cationiques utiles dans le procédé de l'invention sont bien connues de la technique. Ces bases sont par exemple des paraphénylènediamines simples ou des paraphénylènediamines doubles dont un ou plusieurs des groupement amino est substitués par un radical cationique et/ou dont le ou les cycles benzéniques sont substitutés par un ou plusieurs radicaux cationiques

Au sens de la présente invention, on entend par radical cationique, tout radical comportant au moins un atome d'azote quaternisé.

Selon un mode de réalisation, le radical cationique est un radical ammonium quaternaire saturé ou insaturé, appelé Z dans la suite de la description.

A titre d'exemple de radicaux ammonium quaternaire Z, on peut citer les radicaux trialkylammonium.

Selon un autre mode de réalisation, l'atome d'azote quaternisé est inclus dans un cycle.

A titre d'exemple, on peut citer les radicaux Z' tels que les radicaux pyrrolium, imidazolium, pyrazolium, oxazolium, thiazolium, triazolium, pyridinium, pyrimidinium, pyrazinium, oxazinium et triazinium. Les radicaux Z' imidazolium et pyridinium sont particulièrement préférés.

Les bases d'oxydations du type paraphénylènediamine substituées par un ou plusieurs radicaux ammonium quaternaires utiles pour l'invention sont par exemple décrites dans la demande de brevet EP 968 171.

On peut notamment citer :

- le chlorure de [2-(2,5-diamino-phénoxy)-éthyl]-diéthyl-méthyl-ammonium monohydrate ;
- le dichlorure de N,N-bis-(triméthylammonium-propyl)-4-amino-aniline ;
- le chlorure de [4-(4-amino-phénylamino)-pentyl]-diéthyl-méthyl-ammonium ;
- le chlorure de [4-(4-amino-phénylamino)-pentyl]-diéthyl-(2-hydroxyéthyl)-ammonium ;
- le chlorure de [2-(4-amino-phénylamino)-éthyl]-diéthyl-méthyl-ammonium ;
- le chlorure de {2-[(4-aminophényl)-méthyl-amino]-éthyl}-triméthyl-ammonium ;
- le chlorure de [3-(4-amino-phénylamino)-propyl]-triméthyl-ammonium ;
- le chlorure de [2-(4-amino-phénylamino)-propyl]-triméthyl-ammonium ;
- le chlorure de [4-(4-amino-2-méthyl-phénylamino)-pentyl]-diéthyl-méthyl-ammonium ;
- le chlorure de [4-(4-amino-3-méthyl-phénylamino)-pentyl]-diéthyl-méthyl-ammonium ;

et leurs sels d'addition avec un acide.

Parmi ces bases, on préfère plus particulièrement :

- le chlorure de [2-(2,5-diamino-phénoxy)-éthyl]-diéthyl-méthyl-ammonium monohydrate ;
- le chlorure de N,N-bis-(triméthylammonium-propyl)-4-amino-aniline ;
- le chlorure de [4-(4-amino-phénylamino)-pentyl]-diéthyl-méthyl-ammonium ;
- le chlorure de [2-(4-amino-phénylamino)-éthyl]-diéthyl-méthyl-ammonium ;
- le chlorure de {2-[(4-aminophényl)-méthyl-amino]-éthyl}-triméthyl-ammonium ;
- le chlorure de [3-(4-amino-phénylamino)-propyl]-triméthyl-ammonium ;
- le chlorure de [4-(4-amino-phénylamino)-pentyl]-diéthyl-(2-hydroxyéthyl)-ammonium ;

et leurs sels d'addition avec un acide.

On peut aussi citer les bases d'oxydation du type paraphénylènediamine substituée par un ou plusieurs cationiques cycliques décrites dans la demande de brevet EP 928289.

A titre d'exemple, on peut citer

- le bromure de 1-[2-(4-amino-phénylamino)-éthyl]-3-méthyl-3H-imidazol-1-ium ;
- le chlorure de 1-[3-(2,5-diamino-phénoxy)-propyl]-3-méthyl-3H-imidazol-1-ium ;

- le chlorure de 3-[3-(4-amino-phénylamino)-propyl]-1-méthyl-3H-imidazol-1-ium ;
- le chlorure de 3-[3-(4-amino-3-méthyl-phénylamino)-propyl]-1-méthyl-3H-imidazol-1-ium ;
- le chlorure de 3-[3-(4-amino-2-méthyl-phénylamino)-propyl]-1-méthyl-3H-imidazol-1-ium ;
- le chlorure de 3-[3-(4-amino-2-fluoro-phénylamino)-propyl]-1-méthyl-3H-imidazol- 1-ium, monohydrate ;
- le chlorure de 3-[3-(4-amino-2-cyano-phénylamino)-propyl]-1-méthyl-3H-imidazol-1-ium ;
- le chlorure de 1-[2-(4-amino-2-méthoxy-phénylamino)-éthyl]-3-méthyl-3H-imidazol-1-ium ;
- le chlorure de 1-(5-amino-2-hydroxy-benzyl)-3-méthyl-3H-imidazol-1-ium ;
- le chlorure de 1-(5-amino-2-hydroxy-benzyl)-2-méthyl-2H-pyrazol-1-ium ;
- le chlorure de 1-[2-(2,5-diamino-phényl)-éthyl]-3-méthyl-3H-imidazol-1-ium ;
- le chlorure de 3-[2-(2,5-diamino-phényl)-éthyl]-1-méthyl-3H-imidazol-1-ium ;
- le chlorure de 1-{2-[(4-amino-phényl)-éthylamino]-éthyl}-3-méthyl-3H-imidazol-1-ium ;
- le dichlorure de N,N-bis-[2-(3-méthyl-3H-imidazol-1-ium)-éthyl]-4-amino-aniline ;
- le chlorure de 3-[2-(4-amino-phénylamino)-butyl]-1-méthyl-3H-imidazol-1-ium ;
- le chlorure de 1-{[5-amino-2-(2-hydroxy-éthylamino)-phénylcarbamoyl]-méthyl}-3-méthyl-3H-imidazol-1-ium ;
- le bromure de 4-[2-(2,5-diamino-phénoxy)-éthyl]-1,3-diméthyl-3H-imidazol-1-ium ;
- le chlorure de 2-(2,5-diamino-phénoxyméthyl)-1,3-diméthyl-3H-imidazol-1-ium;
- le chlorure de 4-[3-(4-amino-phénylamino)-propyl]-1,3-diméthyl-3H-imidazol-1-ium ;
- le chlorure de 4-[3-(4-amino-3-méthyl-phénylamino)-propyl]-1,3-diméthyl-3H-imidazol-1-ium ;
- le chlorure 4-[(2,5-diamino-phénylcarbamoyl)-méthyl]-1,3-diméthyl-3H-imidazol-1-ium ;
- le chlorure de 4-{2-[2-(2-amino-5-hydroxy-phényl)-acétylamino]-éthyl}-1,3-diméthyl-3H-imidazol-1-ium ;
- le chlorure de 4-[(5-amino-2-hydroxy-benzylcarbamoyl)-méthyl]-1,3-diméthyl-3H-imidazol-1-ium ;
  et leurs sels d'addition avec un acide.

On peut aussi citer le composé suivant :

- chlorure de 1-{[5-amino-2-(2-hydroxyéthylamino)-phénylcarbamoyl]-méthyl}-1,4-diméthyl-pipérazin-1-ium.

Des bases d'oxydation du type paraphénylènediamine substituée par un radical cationique utiles pour l'invention sont aussi des bases doubles paraphénylènediamine substituées par un ou plusieurs radicaux cationiques décrites dans la demande de brevet EP 932602.

A titre d'exemple, on peut citer

- le dichlorure de 1,3-bis-1{3{3'[(4"-amino-3"-méthyl-aniline)-N-propyl]}-3H-imidazol-1-ium}-propane ;
- le dichlorure de 1,3-bis-1{3{3'[(4"-amino-2"-méthyl-aniline)-N-propyl]}-3H-imidazol-1-ium}-propane, monohydrate, diéthanol ;
- le dichlorure de 1,3-bis-1{3{3'[(4"-amino-aniline)-N-propyl]}-3H-imidazol-1-ium}-propane, monohydrate, éthanol ;
- le tétrachlorure de 1,3-bis-{3{3'[(4'amino-aniline)-N-propyl]}-3H-imidazol-1-ium} propane ;
- le dichlorure de 1,3-bis-1{3{3'[(4"-amino-aniline)-N-propyl]}-3H-imidazol-1-ium}-2-propanol, monohydrate ;
- le dibromure de $N_1$,$N_3$-bis-[3-N(4'-amino-aniline)-propyl]-1,1,3,3-tétraméthyl-diammonium 1-3-propane, monohydrate ;
- le dichlorure de 1,4-bis-1{3[3-(2,5-diamino-phénoxy)-propyl]-3H-imidazol-1-ium}- butane, dihydrate ;
- le monochlorure de 1,3-bis-[3-(2,5-diamino-phénoxy)-propyl]-3H-imidazol-1-ium, monohydrate ;
- le dibromure de $N_1$,$N_4$-bis-[3-N-méthyl-N-(4'-amino-aniline)-éthyl]-1,1,4,4-tétraméthyl-diammonium 1-3-propane, monohydrate ;
- le dichlorure de 1,4-bis-1-[3-(5-amino-2-hydroxy-benzyl)-3H-imidazol-1 ium]-butane, monohydrate ;
- le dibromure de 1,3-bis-{[2-(4-amino-aniline)-propyl]-1,1,3,3-tétraméthyl-diammonium-propane ;
- le dichlorure de 1,3-bis-{[4-(4-amino-aniline)-pentyl]-1,1,3,3-tétraméthyl-diammonium-propane ;
- le monochlorure de [4-(4-amino-phénylamino)-pentyl]-(5-amino-2-hydroxy-benzyl)-diéthyl-ammonium ;
- le monochlorure de [2-(4-amino-phénylamino)-propyl]-(5-amino-2-hydroxy-benzyl)-diméthyl-ammonium ;
- le dichlorure de 1,3-bis-1-{3-[3-(2,5-diamino-phénoxy)-propyl]-3H-imidazol-1-ium}-propane, dihydrate ;
- le dichlorure de 1,3-bis-1{3{3'[(4"-amino-aniline)-N-propyl]}-3H-imidazol-1-ium}-propane ;
- le dichlorure de 1,3-bis-1{4{4'(4-[3-(4"-amino-phénylamino)-propyl] }-1,3-diméthyl-3H-imidazol-1-ium}-propane,
- le dichlorure de 1,3-bis-1{4{4'(4-[3-(4"-amino-2"-méthyl-aniline)-propyl] }-1,3-diméthyl-3H-imidazol-1-ium}-propane ;
- le monochlorure de 4-[2-(2,5-diamino-phénoxy)-éthyl]-3-[3-(2,5-diamino-phénoxy)-propyl]-1-méthyl-3-imidazol-1-ium ;
- le monochlorure de 4-[2-(2,5-diamino-phénoxy)-éthyl]-1-[3-(2,5-diamino-phénoxy)-propyl]-3-méthyl-3-imidazol-1-ium ;

et leurs sels d'addition avec un acide.

Parmi ces bases doubles, on préfère plus particulièrement :

- le dichlorure de 1,3-bis-1-{3-{3'-[(4"-amino-aniline)-N-propyl]}-3H-imidazol-1-ium}-propane, monohydrate, éthanol ;
- le monochlorure de 1,3-bis-[3-(2,5-diamino-phénoxy)-propyl]-3H-imidazol-1-ium, monohydrate ;
- le dibromure de $N_1$,$N_4$-bis-[3-N-méthyl-N-(4'-amino-aniline)-éthyl]-1,1,4,4-tétraméthyl-diammonium 1-3-propane, monohydrate ;
- le dichlorure de 1,4-bis-1[3-(5-amino-2-hydroxy-benzyl)-3H-imidazol-1-ium]-butane, monohydrate ;
- le dichlorure de 1,3-bis-1{3{3'[(4"-amino-aniline)-N-propyl]}-3H-imidazol-1-ium}-propane ;

et leurs sels d'addition avec un acide.

D'autres bases d'oxydation du type para-phénylènediamine substituées par un radical cationique sont des paraphénylènediamine à groupement pyrrolidinyle substitués par un ou plusieurs radicaux cationiques. De telles bases sont décrites dans la demande de brevet EP 1 348 695.

A titre d'exemple, on peut citer

| | | | |
|---|---|---|---|
| 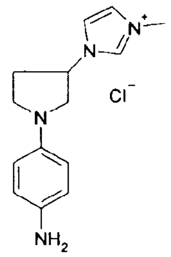 | 3-[1-(4-Amino-phényl)-pyrrolidin-3-yl]-1-méthyl-3H-imidazol-1-ium; chlorure | 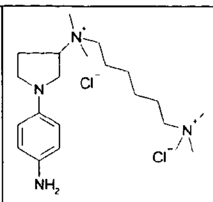 | [1-(4-Amino-phényl)-pyrrolidin-3-yl]-(-triméthylammonium-hexyl)-diméthyl-ammonium; dichlorure |
| 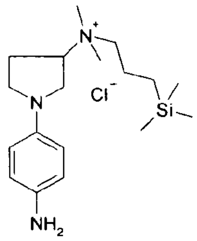 | [1-(4-Amino-phényl)-pyrrolidin-3-yl]-diméthyl-(3-triméthylsilanyl-propyl)-ammonium ; chlorure | 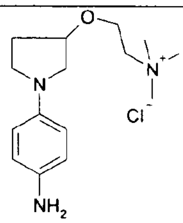 | {2-[1-(4-Amino-phényl)-pyrrolidin-3-yloxy]-ethyl}-triméthyl-ammonium; chlorure |
| 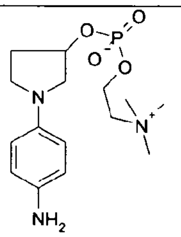 | [1-(4-Amino-phényl)-pyrrolidin-3-yl]-oxophosphorylcholine | 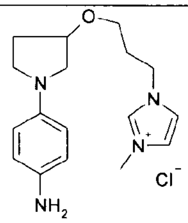 | 3-{3-[1-(4-Amino-phényl)-pyrrolidin-3-yloxy]-propyl}-1-méthyl-3H-imidazol-1-ium ; chlorure |
| 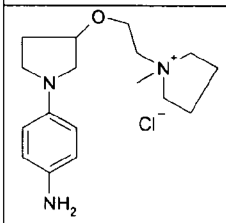 | 1-{2-[1-(4-Amino-phényl)-pyrrolidin-3-yloxy]-ethyl}-1-méthyl-pyrrolidinium; chlorure | 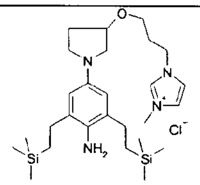 | 3-{3-[1-(5-triméthylsilanylethyl-4-Amino-3-triméthylsilanylethyl -phényl)-pyrrolidin-3-yloxy]-propyl}-1-méthyl-3H-imidazol-1-um ; chlorure |
| 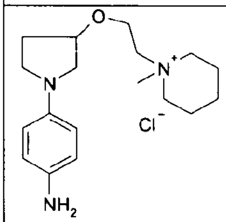 | 1-{2-[1-(4-Amino-phényl)-pyrrolidin-3-yloxy]-ethyl}-1-méthyl-piperidinium; chlorure | 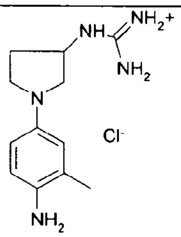 | N-[1-(4-Amino-3-méthyl-phényl)-pyrrolidin-3-yl]-guanidinium ; chlorure |
| 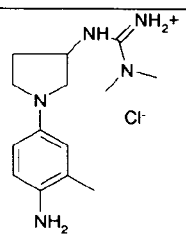 | N'-[1-(4-Amino-3-méthyl-phényl)-pyrrolidin-3-yl]-N,N-diméthyl-guanidinium ; chlorure | 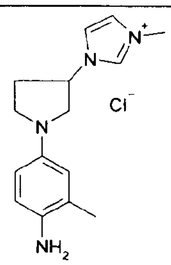 | 3-[1-(4-Amino-3-méthyl-phényl)-pyrrolidin-3-yl]-1-méthyl-3H-imidazol-1-ium; chlorure |

| | | | |
|---|---|---|---|
| 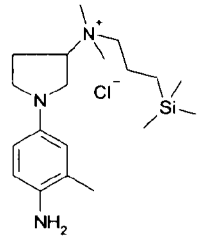 | [1-(4-Amino-3-méthyl-phényl)-pyrrolidin-3-yl]-diméthyl-(3-triméthylsilanyl-propyl ammonium ; chlorure | 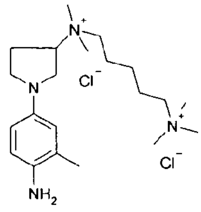 | [1-(4-Amino-3-méthyl-phényl)-pyrrolidin-3-yl]-(-triméthylammonium-hexyl)-diméthyl-ammonium dichlorure |
| 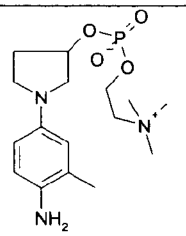 | [1-(4-Amino-3-méthyl-phényl)-pyrrolidin-3-yl]-oxophosphorylcholine | 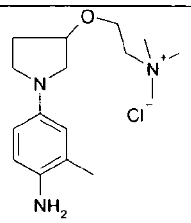 | {2-[1-(4-Amino-3-méthyl-phényl)-pyrrolidin-3-yloxy]-ethyl}-triméthyl-ammonium; chlorure |
| 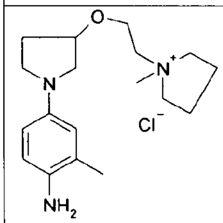 | 1-{2-[1-(4-Amino-3-méthyl-phényl)-pyrrolidin-3-yloxy]-ethyl}-1-méthyl-pyrrolidinium ; chlorure | 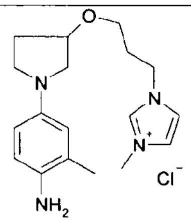 | 3-{3-[1-(4-Amino-3-méthyl-phényl)-pyrrolidin-3-yloxy]-propyl}-1-méthyl-3H-imidazol-1-um ; chlorure |
| 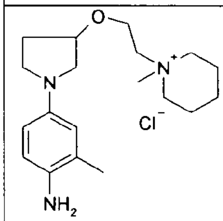 | 1-{2-[1-(4-Amino-3-méthyl-phényl)-pyrrolidin-3-yloxy]-ethyl}-1-méthyl-piperidin | 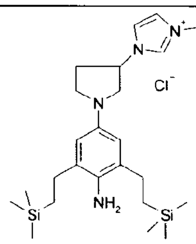 | 3-[1-(5-triméthylsilanylethyl-4-Amino-3-triméthylsilanylethyl-phényl)-pyrrolidin-3-yl]-1-méthyl-3H-imidazol-1-ium; chlorure |
| 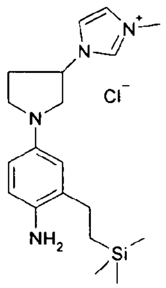 | 3-[1-(4-Amino-3-triméthylsilanylethyl-phényl)-pyrrolidin-3-yl]-1-méthyl-3H-imidazol-1-ium; chlorure | 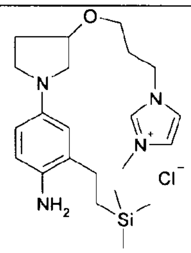 | 3-{3-[1-(4-Amino-3-triméthylsilanylethyl -phényl)-pyrrolidin-3-yloxy]-propyl}-1-méthyl-3H-imidazol-1-um ; chlorure |
| 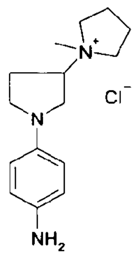 | 1'-(4-Amino-phényl)-1-méthyl-[1,3']bipyrrolidinyl-1-ium; chlorure | 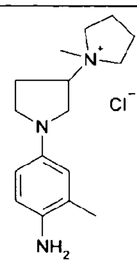 | 1'-(4-Amino-3-méthyl-phényl)-1-méthyl-[1,3']bipyrrolidinyl-1-ium; chlorure |

| | | | |
|---|---|---|---|
| 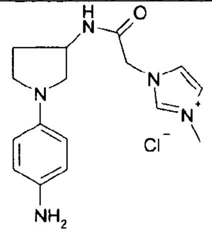 | 3-{[1-(4-Amino-phényl)-pyrrolidin-3-ylcarbamoyl]-méthyl}-1-méthyl-3H-imidazol-1-ium ; chlorure | 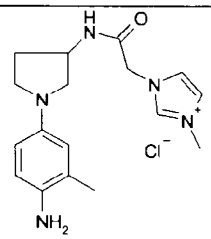 | 3-{[1-(4-Amino-3-méthyl-phényl)-pyrrolidin-3-ylcarbamoyl]-méthyl}-1-méthyl-3H- imidazol-1-ium ; chlorure |
| 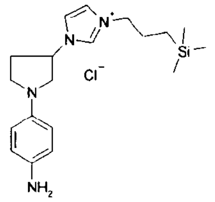 | 3-[1-(4-Amino-phényl)-pyrrolidin-3-yl]-1-(3-triméthylsilanyl-propyl)-3H-imidazol-1-ium ; chlorure | 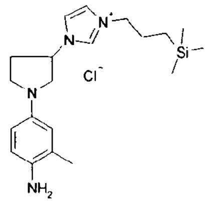 | 3-[1-(4-Amino-3-méthyl-phényl)-pyrrolidin-3-yl]-1-(3-triméthylsilanyl-propyl)-3H-imidazol-1-ium ; chlorure |
| 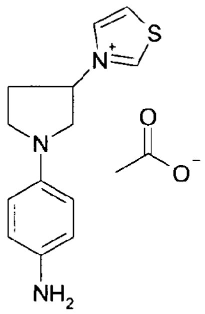 | Acétate de 3-[1-(4-Amino-phenyl)-pyrrolidin-3-yl]-thiazol-3-ium | 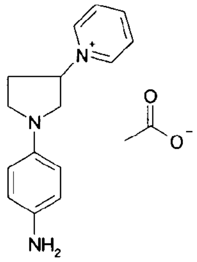 | Acétate de 1-[1-(4-aminophenyl)pyrrolidin-3-yl]pyridinium |
| 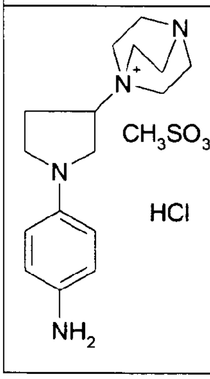 | 1-[1-(4-Amino-phenyl)-pyrrolidin-3-yl]-4-aza-1-azonia-bicyclo[2.2.2] octane ;méthanesulfonate ; chlorhydrate | | |

Parmi ces bases d'oxydation, les composés suivants sont particulièrement préférés :

- N'-[1-(4-Amino-phényl)-pyrrolidin-3-yl]-N,N-diméthyl-guanidinium ; chlorure
- N-[1-(4-Amino-phényl)-pyrrolidin-3-yl]-guanidinium ; chlorure
- 3-[1-(4-Amino-phényl)-pyrrolidin-3-yl]-1-méthyl-3H-imidazol-1-ium; chlorure
- [1-(4-Amino-phényl)-pyrrolidin-3-yl]-diméthyl-(3-triméthylsilanyl-propyl)-ammonium ; chlorure
- N'-[1-(4-Amino-3-méthyl-phényl)-pyrrolidin-3-yl]-N,N-diméthyl-guanidinium ; chlorure
- N-[1-(4-Amino-3-méthyl-phényl)-pyrrolidin-3-yl]-guanidinium ; chlorure
- 3-[1-(4-Amino-3-méthyl-phényl)-pyrrolidin-3-yl]-1-méthyl-3H-imidazol-1-ium; chlorure
- [1-(4-amino 3-méthyl phényl) pyrrolidin 3 -yl] diméthyl (3-triméthylsilanyl propyl ammonium ; chlorure ;
- 3-[1-(4-Amino-phényl)-pyrrolidin-3-yl]-1-(3-triméthylsilanyl-propyl)-3H-imidazol-1-ium ; chlorure
- 3-[1-(4-Amino-3-méthyl-phényl)-pyrrolidin-3-yl]-1-(3-triméthylsilanyl-propyl)-3H-imidazol-1-ium ; chlorure
- 1'-(4-Amino-phényl)-1-méthyl-[1,3']bipyrrolidinyl-1-ium; chlorure
- 1'-(4-Amino-3-méthyl-phényl)-1-méthyl-[1,3']bipyrrolidinyl-1-ium; chlorure

- 3-{[1-(4-Amino-phényl)-pyrrolidin-3-ylcarbamoyl]-méthyl}-1-méthyl-3H-imidazol-1-ium ; chlorure
- 3-{[1-(4-Amino-3-méthyl-phényl)-pyrrolidin-3-ylcarbamoyl]-méthyl}-1-méthyl-3H- imidazol-1-ium ; chlorure
- 1'-(4-amino-phényl)-1-méthyl-[1,3']bipyrrolidinyl-1-ium, chlorure.

On peut aussi citer à titre de bases d'oxydation du type para-phénylènediamine substituées par un ou plusieurs radicaux ammoniums quaternaires, des bases bis-paraphénylènediamine à groupement pyrrolidinyle substitué par un ou plusieurs radicaux ammoniums quaternaires telles que décrites dans la demande de brevet EP 1 396 486.

A titre d'exemple, on peut citer

| Structure | Nomenclature |
|---|---|
| 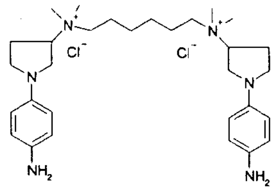 | N,N'-bis[1-(4-aminophenyl)pyrrolidin-3-yl]-N,N,N',N'-tetramethylhexane-1,6-diaminium dichlorure |
| 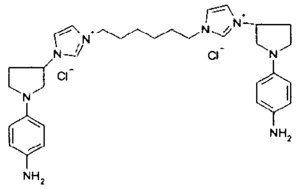 | 3-[1-(4-aminophenyl)pyrrolidin-3-yl]-1-(6-{1-[1-(4-aminophenyl)pyrrolidin-3-yl]-1H-imidazol-3-ium-3-yl}hexyl)-1H-imidazol-3-ium dichlorure |
| 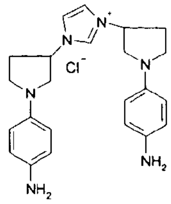 | 1,3-bis[1-(4-aminophenyl)pyrrolidin-3-yl]-1H-imidazol-3-ium chlorure |

| Structure | Nomenclature |
|---|---|
| 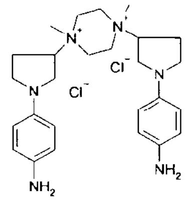 | 1,4-bis[1-(4-aminophenyl)pyrrolidin-3-yl]-1,4-dimethylpiperazinediium dichlorure |
| 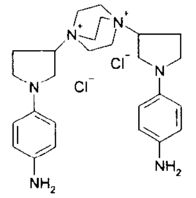 | 1,4-bis[1-(4-aminophenyl)pyrrolidin-3-yl]-1,4-diazoniabicyclo[2.2.2]octane dichlorure |
| 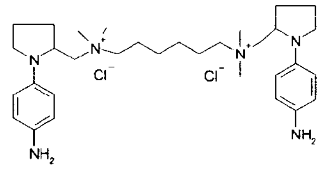 | N,N'-bis{[1-(4-aminophenyl)pyrrolidin-2-yl]methyl}-N,N,N',N'-tetramethylhexane-1,6-diaminium dichlorure |
| 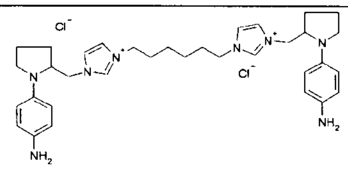 | 3-{[1-(4-aminophenyl)pyrrolidin-2-yl]methyl}-1-[6-(1-{[1-(4-aminophenyl)pyrrolidin-2-yl]methyl}-1H-imidazol-3-ium-3-yl)hexyl]-1H-imidazol-3-ium dichlorure |
| 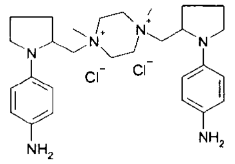 | 1,4-bis{[1-(4-aminophenyl)pyrrolidin-2-yl]methyl}-1,4-dimethylpiperazinediium dichlorure |
| 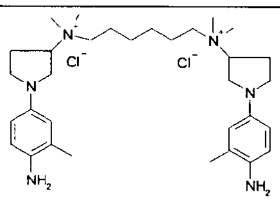 | N,N'-bis[1-(4-amino-3-methylphenyl)pyrrolidin-3-yl]-N,N,N',N'-tetramethylhexane-1,6-diaminium dichlorure |
| 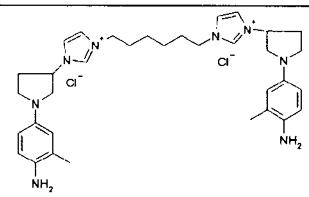 | 3-[1-(4-amino-3-methylphenyl)pyrrolidin-3-yl]-1-(6-{1-[1-(4-amino-3-methylphenyl)pyrrolidin-3-yl]-1H-imidazol-3-ium-3-yl}hexyl)-1H-imidazol-3-ium dichlorure |
| 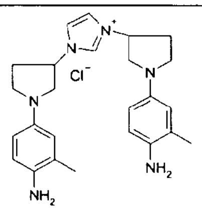 | 1,3-bis[1-(4-amino-3-methylphenyl)pyrrolidin-3-yl]-1H-imidazol-3-ium chlorure |

| Structure | Nomenclature |
|---|---|
| 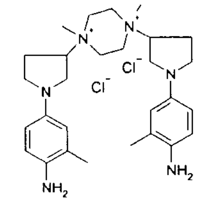 | 1,4-bis[1-(4-amino-3-methylphenyl)pyrrolidin-3-yl]-1,4-dimethylpiperazinediium dichlorure |
| 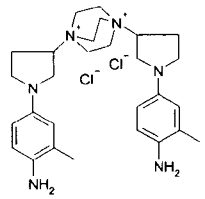 | 1,4-bis[1-(4-amino-3-methylphenyl)pyrrolidin-3-yl]-1,4-diazoniabicyclo[2.2.2]octane dichlorure |
| 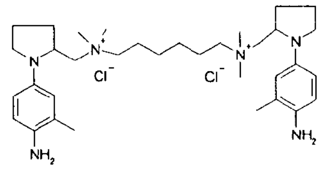 | N,N'-bis{[1-(4-amino-3-methylphenyl)pyrrolidin-2-yl]methyl}-N,N,N',N'-tetramethylhexane-1,6-diaminium dichlorure |
| 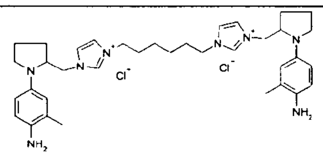 | 3-{[1-(4-amino-3-methylphenyl)pyrrolidin-2-yl]methyl}-1-[6-(1-{[1-(4-amino-3-methylphenyl)pyrrolidin-2-yl]methyl}-1H-imidazol-3-ium-3-yl)hexyl]-1H-imidazol-3-ium dichlorure |
| 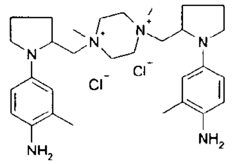 | 1,4-bis{[1-(4-amino-3-methylphenyl)pyrrolidin-2-yl]methyl}-1,4-dimethylpiperazinediium dichlorure |
| 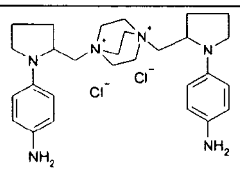 | 1,4-bis{[1-(4-aminophenyl)pyrrolidin-2-yl]methyl}-1,4-diazoniabicyclo[2.2.2]octane dichlorure |
| 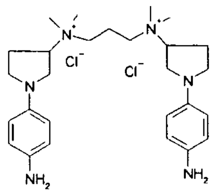 | N,N'-bis[1-(4-aminophenyl)pyrrolidin-3-yl]-N,N,N',N'-tetramethylpropane-1,3-diaminium dichlorure |
| 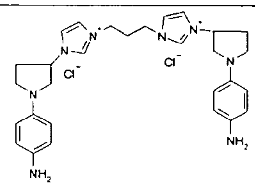 | 3-[1-(4-aminophenyl)pyrrolidin-3-yl]-1-(3-{1-[1-(4-aminophenyl)pyrrolidin-3-yl]-1H-imidazol-3-ium-3-yl}propyl)-1H-imidazol-3-ium dichlorure |

| Structure | Nomenclature |
|---|---|
| 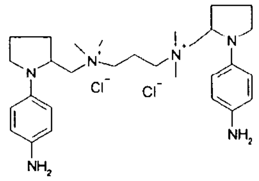 | N,N'-bis{[1-(4-aminophenyl)pyrrolidin-2-yl]methyl}-N,N,N',N'-tetramethylpropane-1,3-diaminium dichlorure |
| 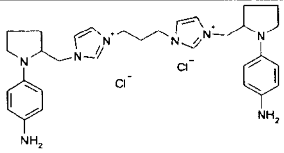 | 3-{[1-(4-aminophenyl)pyrrolidin-2-yl]methyl}-1-[3-(1-{[1-(4-aminophenyl)pyrrolidin-2-yl]methyl}-1H-imidazol-3-ium-3-yl)propyl]-1H-imidazol-3-ium dichlorure |
| 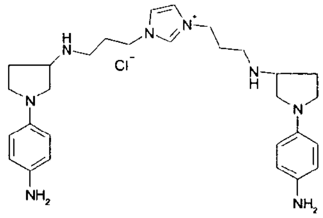 | 1,3-bis(3-{[1-(4-aminophenyl)pyrrolidin-3-yl]amino}propyl)-1H-imidazol-3-ium chlorure |
| 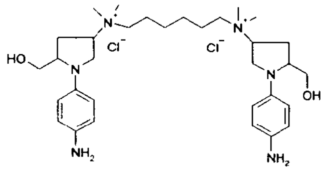 | N,N'-bis[1-(4-aminophenyl)-5-(hydroxymethyl)pyrrolidin-3-yl]-N,N,N',N'-tetramethylhexane-1,6-diaminium dichlorure |
| 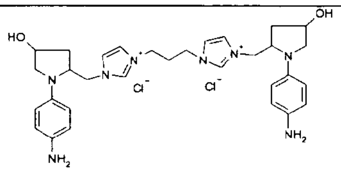 | 3-{[1-(4-aminophenyl)-4-hydroxypyrrolidin-2-yl]methyl}-1-[3-(1-{[1-(4-aminophenyl)-4-hydroxypyrrolidin-2-yl]methyl}-1H-imidazol-3-ium-3-yl)propyl]-1H-imidazol-3-ium dichlorure |
| 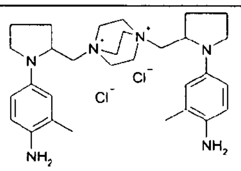 | 1,4-bis{[1-(4-amino-3-methylphenyl)pyrrolidin-2-yl]methyl}-1,4-diazoniabicyclo[2.2.2]octane dichlorure |
| 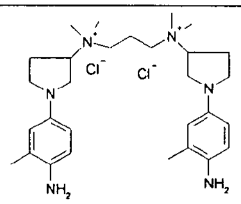 | N,N'-bis[1-(4-amino-3-methylphenyl)pyrrolidin-3-yl]-N,N,N',N'-tetramethylpropane-1,3-diaminium dichlorure |
| 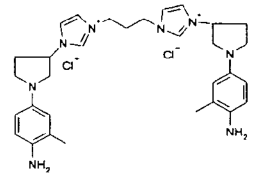 | 3-[1-(4-amino-3-methylphenyl)pyrrolidin-3-yl]-1-(3-{1-[1-(4-amino-3-methylphenyl)pyrrolidin-3-yl]-1H-imidazol-3-ium-3-yl}propyl)-1H-imidazol-3-ium dichlorure |

| Structure | Nomenclature |
|---|---|
| 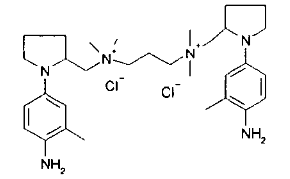 | N,N'-bis{[1-(4-amino-3-methylphenyl)pyrrolidin-2-yl]methyl}-N,N,N',N'-tetramethylpropane-1,3-diaminium dichlorure |
| 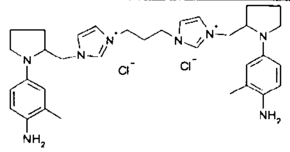 | 3-{[1-(4-amino-3-methylphenyl)pyrrolidin-2-yl]methyl}-1-[3-(1-{[1-(4-amino-3-methylphenyl)pyrrolidin-2-yl]methyl}-1H-imidazol-3-ium-3-yl)propyl]-1H-imidazol-3-ium dichlorure |
| 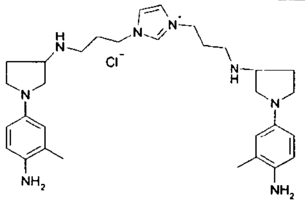 | 1,3-bis(3-{[1-(4-amino-3-methylphenyl)pyrrolidin-3-yl]amino}propyl)-1H-imidazol-3-ium chlorure |
| 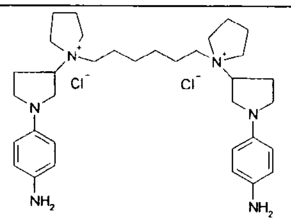 | N,N'-bis[1-(4-aminophenyl)pyrrolidin-3-yl]-N,N',dipyrrolidinehexane-1,6-diaminium dichlorure |
| 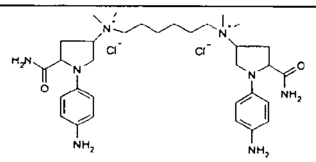 | N,N'-bis[1-(4-aminophenyl)pyrrolidin-5-amido-3-yl]-N,N,N',N'-tetramethylhexane-1,6-diaminium dichlorure |
| 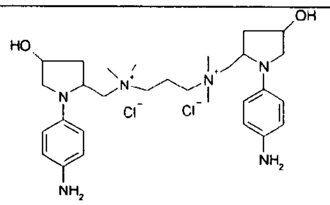 | N,N'-bis{[1-(4-aminophenyl)-4-hydroxypyrrolidin-2-yl]methyl}-N,N,N',N'-tetramethylpropane-1,3-diaminium dichlorure |

Selon un mode de réalisation préféré, les bases d'oxydation du type paraphénylènediamine utiles pour l'invention sont des bases doubles dicationiques.

Il est à noter que la nature du ou des contre-ions et des éventuels résidus de solvatation n'est pas critique dans l'invention.

La base préférée dans le cadre de l'invention est choisi parmi les composés :

- le dichlorure de 1,3-bis-1{3{3'[(4"-amino-aniline)-N-propyl]}-3H-imidazol-1-ium}-propane, monohydrate, éthanol ;
- le térachlorure de 1,3-bis-{3{3'[(4'amino-aniline)-N-propyl]}-3H-imidazol-1-ium} propane

La composition de coloration utile dans la présente invention peut comprendre une ou plusieurs autres bases d'oxydation différentes de celles décrites précédemment. A titre de bases additionnelles, on peut citer les paraphénylènediamines non cationiques, les bases doubles para-phénylènediamine non cationiques, les bases doubles para-aminophénols, les para-aminophénols, les ortho aminophénols et les bases d'oxydation hétérocycliques.

Parmi les paraphénylènediamines non cationiques, on peut notamment citer les composés de formule (1) suivante et leurs sels d'addition avec un acide :

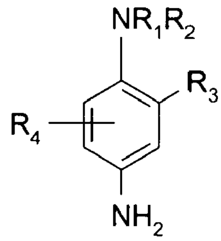

(I)

dans laquelle :

- $R_1$ représente un atome d'hydrogène, un radical alkyle en $C_1$-$C_4$, monohydroxyalkyle en $C_1$-$C_4$, polyhydroxyalkyle en $C_2$-$C_4$, alcoxy($C_1$-$C_4$)alkyle($C_1$-$C_4$), alkyle en $C_1$-$C_4$ substitué par un groupement azoté, phényle ou 4'-aminophényle ;
- $R_2$ représente un atome d'hydrogène, un radical alkyle en $C_1$-$C_4$, monohydroxyalkyle en $C_1$-$C_4$, polyhydroxyalkyle en $C_2$-$C_4$, alcoxy($C_1$-$C_4$)alkyle($C_1$-$C_4$) ou alkyle en $C_1$-$C_4$ substitué par un groupement azoté ;
- $R_3$ représente un atome d'hydrogène, un atome d'halogène tel qu'un atome de chlore, de brome, d'iode ou de fluor, un radical alkyle en $C_1$-$C_4$, monohydroxyalkyle en $C_1$-$C_4$, hydroxyalcoxy en $C_1$-$C_4$, acétylaminoalcoxy en $C_1$-$C_4$, mésylaminoalcoxy en $C_1$-$C_4$

ou carbamoylaminoalcoxy en $C_1$-$C_4$,

- $R_4$ représente un atome d'hydrogène, d'halogène ou un radical alkyle en $C_{1}$-$C_4$,

ainsi que les bases d'oxydation dans lesquelles R1 et R2 forment ensemble un groupement pyrrolidine, substitué ou non substitué.

Parmi les paraphénylènediamines de formule (I) ci-dessus, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl) amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les bases doubles non cationiques utilisables dans les compositions de coloration d'oxydation utiles dans la présente invention, on peut notamment citer les composés répondant à la formule (II) suivante, et leurs sels d'addition avec un acide :

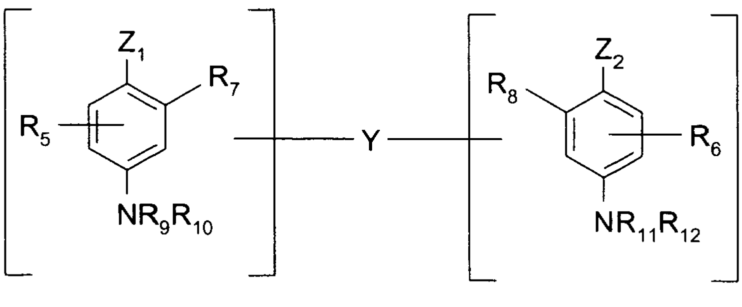

(II)

dans laquelle :

- $Z_1$ et $Z_2$, identiques ou différents, représentent un radical hydroxyle ou $-NH_2$ pouvant être substitué par un radical alkyle en $C_1$-$C_4$ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en $C_1$-$C_6$ ;
- $R_5$ et $R_6$ représentent un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$

ou un bras de liaison Y ;

- $R_7$, $R_8$, $R_9$, $R_{10}$ $R_{11}$ et $R_{12}$, identiques ou différents, représentent un atome d'hydrogène, un bras de liaison Y ou un radical alkyle en $C_1$-$C_4$ ; ou R9 avec R10 ou R11 avec R12 forment ensemble un radical pyrrolidinyle, étant entendu que les composés de formule (II) ne comportent qu'un seul bras de liaison Y par molécule.

Parmi les groupements azotés de la formule (II) ci-dessus, on peut citer notamment les radicaux amino, monoalkyl($C_1$-$C_4$)amino, dialkyl($C_1$-$C_4$)amino, trialkyl($C_1$-$C_4$)amino, monohydroxyalkyl($C_1$-$C_4$)amino.

Parmi les bases doubles de formules (II) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,6-dioxaoctane, N,N'-bis[1-(4-aminophenyl) pyrrolidin-3-yl]butane-1,2-diamine, 1,3-bis{[1-(4-aminophenyl)pyrrolidin-2-yl]methoxy}-propane, N,N'-bis[1-(4-aminophenyl)pyrrolidin-3-yl]ethane-1,2-diamine, et leurs sels d'addition avec un acide.

Parmi les para-aminophénols utilisables à titre de bases d'oxydation dans les compositions de coloration d'oxydation utiles dans la présente invention, on peut notamment citer les composés répondant à la formule (III) suivante, et leurs sels d'addition avec un acide :

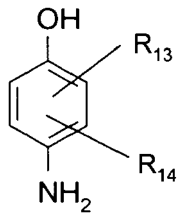

(III)

dans laquelle :

- $R_{13}$ représente un atome d'hydrogène ou d'halogène, un radical alkyle en $C_1$-$C_4$, monohydroxyalkyle en $C_1$-$C_4$, alcoxy($C_1$-$C_4$)alkyle($C_1$-$C_4$), aminoalkyle en $C_1$-$C_4$ ou hydroxyalkyl($C_1$-$C_4$)aminoalkyle en $C_1$-$C_4$,
- $R_{14}$ représente un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxy($C_1$-$C_4$)alkyle($C_1$-$C_4$), étant entendu qu'au moins un des radicaux $R_{13}$ ou $R_{14}$ représente un atome d'hydrogène.

A titre de para-aminophénols on peut aussi citer le 4 amino,6 [(5'-amino-2' hydroxy 3'methylphenyl)methyl] 2 méthylphenol et le bis 5 amino 2'hydroxyphènyl méthane et leurs sels d'addition avec un acide.

Parmi les para-aminophénols de formule (III) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les orthoaminophénols utilisables à titre de bases d'oxydation dans les compositions de coloration d'oxydation utile dans la présente invention, on peut plus particulièrement citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques utilisables à titre de bases d'oxydation dans les compositions de coloration

d'oxydation utiles dans la présente invention, on peut plus particulièrement citer les dérivés pyridiniques, les dérivés pyrimidiniques, les dérivés pyrazoliques, les dérivés pyrazolo-pyrimidiniques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-333 495 ou demandes de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et leurs sels d'addition avec un acide.

Parmi les dérivés pyrazoliques, on peut plus particulièrement citer les composés diaminopyrazoles tels que les 4,5 diaminopyrazoles ou les 3,4-diaminopyrazoles, notamment ceux décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl) amino 1-méthyl pyrazole, et leurs sels d'addition avec un acide.

On peut aussi citer le 4,5 diamino 1β hydroxyéthyl pyrazole et le 4,5 diamino 1β méthoxyéthyl pyrazole et ses sels d'addition avec un acide.

Parmi les dérivés pyrazolo-pyrimidiniques, on peut plus particulièrement citer les pyrazolo-[1,5-a]-pyrimidines de formule (IV) suivante, leurs sels d'addition avec un acide
ou avec une base et leurs formes tautomères, lorsqu'il existe un équilibre tautomérique :

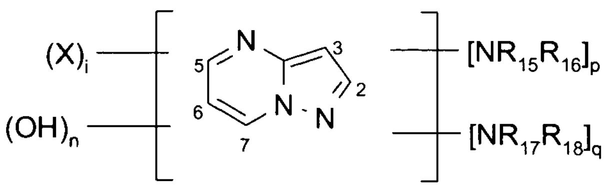

(IV)

dans laquelle :

- $R_{15}$, $R_{16}$, $R_{17}$ et $R_{18}$, identiques ou différents désignent, un atome d'hydrogène, un radical alkyle en $C_1$-$C_4$, un radical aryle, un radical hydroxyalkyle en $C_1$-$C_4$, un radical polyhydroxyalkyle en $C_2$-$C_4$, un radical ($C_1$-$C_4$)alcoxy alkyle en $C_1$-$C_4$, un radical aminoalkyle en $C_1$-$C_4$ (l'amine pouvant être protégée par un radical acétyle, uréido ou sulfonyle), un radical ($C_1$-$C_4$)alkylamino alkyle en $C_1$-$C_4$, un radical di-[(C1-$C_4$)alkyll amino alkyle en $C_1$-$C_4$ (les radicaux dialkyles pouvant former un cycle carboné ou un hétérocycle à 5 ou 6 chaînons), un radical hydroxy($C_1$-$C_4$) alkyl- ou di-[hydroxy($C_1$-$C_4$) alkyl]-amino alkyle en $C_1$-$C_4$;
- les radicaux X désignent , identiques ou différents, un atome d'hydrogène, un radical alkyle en $C_1$-$C_4$, un radical aryle, un radical hydroxyalkyle en $C_1$-$C_4$, un radical polyhydroxyalkyle en $C_2$-$C_4$, un radical amino alkyle en $C_1$-$C_4$, un radical ($C_1$-$C_4$)alkyl amino alkyle en $C_1$-$C_4$, un radical di-[($C_1$-$C_4$)alkyl] amino alkyle en $C_1$-$C_4$ (les dialkyles pouvant former un cycle carboné ou un hétérocycle à 5 ou 6 chaînons), un radical hydroxy($C_1$-C4)alkyl ou di-[hydroxy ($C_1$-$C_4$)alkyl]amino alkyle en $C_1$-$C_4$, un radical amino, un radical ($C_1$-$C_4$)alkyl- ou di-[($C_1$-$C_4$)alkyl]-amino ; un atome d'halogène, un groupe acide carboxylique, un groupe acide sulfonique ;
- i vaut 0, 1, 2 ou 3 ; p vaut 0 ou 1 ; q vaut 0 ou 1 ; n vaut 0 ou 1 ; sous réserve que la somme p + q est différente de 0 ;
- lorsque p + q est égal à 2, alors n vaut 0 et les groupes $NR_{15}R_{16}$ et $NR_{17}R_{18}$ occupent les positions (2,3) ; (5,6) ; (6,7) ; (3,5) ou (3,7) ;
- lorsque p + q est égal à 1 alors n vaut 1 et le groupe $NR_{15}R_{16}$ (ou $NR_{17}R_{18}$) et le groupe OH occupent les positions (2,3) ; (5,6) ; (6,7) ; (3,5) ou (3,7) ;

Parmi les pyrazolo-[1,5-a]-pyrimidines de formule (IV) ci-dessus on peut notamment citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol ; le 2-(7-amino pyrazolo-[1,5-a]-pyrimidin-3-ylamino)-éthanol ; le 2-[(3-Amino-pyrazolo[1,5-a]pyrimidin-7-yl)-(2-hydroxy-éthyl)-amino]-éthanol ; le 2-[(7-Amino-pyrazolo[1,5-a]pyrimidin-3-yl)-(2-hydroxy-éthyl)-amino]-éthanol ; la 5,6-diméthyl pyrazolo-[1,5-a]-pyrimidine-3,7-diamine ; la 2,6-diméthyl pyrazolo-[1,5-a]-pyrimidine-3,7-diamine ; la 2, 5, N 7, N 7-tetraméthyl pyrazolo-[1,5-a]-pyrimidine-3,7-diamine ; et leurs sels d'addition et leurs formes tautomères, lorsqu'il existe un équilibre tautomérique.

La ou les bases d'oxydation cationiques ou non cationiques présentes dans la composition de l'invention sont en général présentes chacune en quantité comprise entre 0,001 et 10 % en poids environ du poids total de la composition tinctoriale, de préférence entre 0,005 et 6 %.

La composition de coloration d'oxydation utile dans la présente invention peut contenir un ou plusieurs coupleurs conventionnellement utilisés pour la teinture de fibres kératiniques. Parmi ces coupleurs, on peut notamment citer les méta-phénylènediamines, les méta-aminophénols, les méta-diphénols, les coupleurs naphtaléniques, les coupleurs hétérocycliques et leur sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(ß-hydroxyéthyloxy) benzène, le 2-amino 4-(ß-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6-hydroxy benzomorpholine la 3,5-diamino-2,6-diméthoxypyridine, le 1-N-(ß-hydroxyéthyl)amino-3,4-méthylène dioxybenzène, le 2,6-bis-(β-hydroxyéthylamino)toluène et leurs sels d'addition avec un acide.

Selon un mode de réalisation particulier, le coupleur est un coupleur méta-phénylènediamine dont au moins un des groupements amino est primaire (-NH2).

Selon ce dernier mode de réalisation, le coupleur méta-phénylènediamine est choisi parmi la métaphénylène diamine, le 2,4-diamino phénoxyéthanol, le 1-méthoxy 2-amino 4-(2'-hydroxy éthylamino)benzène, la 4-fluoro-6-methylbenzene-1,3-diamine, le 1-amino 3-NN bis β hydroxyéthylaminobenzène, le 1,3 bis (2,4-diaminophenoxy)propane, le 1-β-aminoéthyloxy-2,4-diaminobenzène, le 1-β-hydroxyéthyl-2,4-diaminobenzène, l'acide (2,4-diaminophénoxy)acétique, le 4,6-bis (β-hydroxyéthoxy)-1,3-diaminobenzène, le 2,4-diamino 5-méthyléthoxybenzène, le 2,4-diamino 5-β-hydroxyéthytoxytotuène, le 2,4-diméthoxy 1,3-diaminobenzène ou leurs sels d'addition.

Dans la composition de coloration d'oxydation, le ou les coupleurs sont chacun généralement présents en quantité comprise entre 0,001 et 10 % en poids environ du poids total de la composition tinctoriale, de préférence entre 0,005 et 6 %.

D'une manière générale, les sels d'addition avec un acide utilisables dans le cadre des compositions de coloration d'oxydation (bases d'oxydation et coupleurs) sont notamment choisis parmi les chlorhydrates, les bromhydrates, les sulfates et les tartrates, les lactates et les acétates.

La composition de coloration d'oxydation peut de plus contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu approprié pour la coloration d'oxydation appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en $C_1$-$C_4$, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition de coloration d'oxydation peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante :

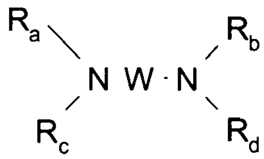

(II)

dans laquelle W est un reste propylène éventuellement substitué par un groupement hydroxyle ou un radical alkyle en $C_1$-$C_4$ ; $R_a$, $R_b$, $R_c$ et $R_d$, identiques ou différents, représentent un atome d'hydrogène, un radical alkyle en $C_1$-$C_4$ ou hydroxyalkyle en $C_1$-$C_4$.

La composition de coloration d'oxydation utile dans le procédé de l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'étape de coloration d'oxydation est en général mise en oeuvre en présence d'un agent oxydant. Cet agent oxydant, appliqué sur les fibres kératiniques en présence de la composition de coloration pendant un temps suffisant permet de développer la coloration désirée. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition de coloration d'oxydation est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu, auquel on peut également ajouter au moins un colorant direct tel que défini ci-dessus, est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition de coloration d'oxydation qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

Le temps nécessaire au développement de la coloration est généralement compris entre 1 et 60 minutes et encore plus précisément 5 et 40 minutes.

La température à laquelle cette étape est réalisée varie en général entre 20°C et 80°C.

Une fois l'étape a) de coloration réalisée, éventuellement suivie d'un rinçage, on effectue éventuellement un lavage des fibres kératiniques au moyen d'une composition lavante (A) comprenant au moins un tensioactif détergent anionique choisi parmi les alkyléthers sulfate et/ou les alkyl sulfate, après quoi on rince lesdites fibres et éventuellement

on les sèche ou on les laisse sécher.

Il est à noter que les parties alkyle des alkyléthers sulfate et/ou les alkyl sulfate listés ci-dessus comprennent avantageusement de 6 à 24 atomes de carbone.

Habituellement, la teneur en tensioactif détergent anionique dans cette composition est comprise entre 4 à 50% en poids par rapport au poids de la composition lavante (A) et de préférence de 6 à 30% en poids par rapport au poids de la composition lavante (A).

La composition lavante (A) peut aussi comprendre un ou plusieurs tensioactifs non ioniques, amphotères ou zwittérioniques, anioniques doux.

Plus particulièrement, à titre de tensioactif non ionique, on peut citer :

- les alcools gras, oxyalkylénés ou polyglycérolés ;
- les alkylphénols dont la chaîne alkyle est en $C_8$-$C_{18}$, oxyalkylénés ;
- les amides gras oxyalkylénés ou polyglycérolés ;
- les amines grasses oxyalkylénées ;
- les huiles végétales oxyalkylénées ;
- les esters d'acides gras du sorbitan, éventuellement oxyalkylénés ;
- les esters d'acides gras du sucrose, éventuellement oxyalkylénés ;
- les esters d'acides gras du polyéthylèneglycol ;
- les alkylpolyglycosides ;
- les dérivés de N-alkyl glucamine ;
- les oxydes d'amines tels que les oxydes d'alkyl ($C_{10}$ - $C_{14}$) amines ou les oxydes de N-acylaminopropylmorpholine ;
- les copolymères d'oxyde d'éthylène et de propylène.

Par chaîne grasse, on entend une chaîne hydrocarbonée comprenant 6 à 30 atomes de carbone, de préférence de 8 à 24 atomes de carbone, linéaire ou ramifiée, saturée ou non.

Plus particulièrement, le nombre moyen de motifs oxyalkylénés est compris entre 2 et 30 motifs. De préférence, il s'agit de motifs oxyéthylénés, oxypropylénés ou leurs mélanges.

En ce qui concerne les tensioactifs polyglycérolés, ils comportent de préférence en moyenne 1 à 5 groupements glycérol et en particulier 1,5 à 4.

Pour ce qui a trait aux tensioactifs amphotères ou zwittérioniques, on peut mentionner sans avoir l'intention de s'y limiter, les dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) betaïnes ou les alkyl ($C_8$-$C_{20}$) amidoalkyl ($C_1$-$C_6$) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

$$R_2\text{-CONHCH}_2\text{CH}_2\text{-N}(R_3)(R_4)(\text{CH}_2\text{COO}^-)$$

dans laquelle : $R_2$ désigne un radical alkyle d'un acide $R_2$-COOH présent dans l'huile de coprah hydrolysée, un radical heptyle, nonyle ou undécyle, $R_3$ désigne un groupement bêta-hydroxyéthyle et $R_4$ un groupement carboxyméthyle ; et

$$R_2'\text{-CONHCH}_2\text{CH}_2\text{-N(B)(C)}$$

dans laquelle :
B représente $-CH_2CH_2OX'$, C représente $-(CH_2)_z-Y'$, avec z = 1 ou 2,
X' désigne le groupement $-CH_2CH_2-COOH$ ou un atome d'hydrogène
Y' désigne -COOH ou le radical $-CH_2-CHOH-SO_3H$
$R_2'$ désigne un radical alkyle d'un acide $R_9$-COOH présent dans l'huile de coprah ou dans l'huile de lin hydrolysée, un radical alkyle, notamment en $C_7$, $C_9$, $C_{11}$ ou $C_{13}$, un radical alkyle en $C_{17}$ et sa forme iso, un radical $C_{17}$ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid, disodium cocoamphocarboxy ethyl hydroxypropyl sulfonate. A titre d'exemple on peut citer le cocoamphodiacetate commercialisé sous la

dénomination commerciale MIRANOL® C2M concentré par la société Rhodia Chimie.

On peut aussi citer les dérivés de formule $RCONH(CH_2)_nN(Y_2X_1)(Y_1-X_2)$, formule dans laquelle :

- $Y_2$ et $Y_1$ étant des radicaux divalents, identiques ou différents, choisis parmi les radicaux alkylènes linéaires ou ramifiés en $C_1$-$C_6$ éventuellement substitués par un ou plusieurs radicaux hydroxyles,
- - $X_1$ et $X_2$ identiques ou différents hydrocarbonés en $C_1$-$C_6$ éventuellement interrompus par un ou plusieurs hétéroatomes et porteurs d'au moins une fonction sel d'acide choisie parmi les groupements carboxylate, sulfonate, sulfate, phosphate ou phosphonate,
- n désignant un nombre entier allant de 1 à 6

En ce qui concerne les tensioactifs anioniques doux, on peut citer notamment les composés de formules suivantes, ainsi que leurs mélanges :

- les acides alkyl éther carboxyliques polyoxyalkylénés,
- les acides alkylaryl éther carboxyliques polyoxyalkylénés,
- les acides alkylamido éther carboxyliques polyoxyalkylénés en particulier ceux comportant 2 à 50 groupements oxyde d'éthylène,
- les acides d'alkyl D galactoside uroniques
- les acylsarcosinates, les acylglutamates ;
- les esters d'alkylpolyglycosides carboxyliques ;
- les sels d'acides gras.

Tout particulièrement, on utilise des acides alkyl éther carboxyliques polyoxyalkylénés comme par exemple l'acide lauryl éther carboxylique (4,5 OE) commercialisé par exemple sous la dénomination AKYPO RLM 45 CA de KAO.

Lorsqu'ils sont présents, la teneur en tensioactifs anioniques doux, amphotères et/ou zwittérionique représente de 4 à 50 % en poids par rapport au poids total des tensioactifs présents dans la première composition lavante (A).

La composition lavante (A) peut de plus comprendre les additifs classiques dans le domaine comme par exemple ceux choisis parmi la liste non exhaustive tels que les agents réducteurs, les agents oxydants, les séquestrants, les adoucissants, les agents anti-mousse, les agents hydratants, les agents émollients, les agents alcalinisants, les plastifiants, les filtres solaires, les colorants directs ou d'oxydation, les pigments, les charges minérales, les argiles, les minéraux colloïdaux, les nacres, les agents nacrant, les parfums, les peptisants, les conservateurs, les polymères fixant ou non, les protéines, les vitamines, les agents antipelliculaires, les alcools aliphatiques ou aromatiques, et plus particulièrement l'éthanol, l'alcool benzylique, les polyols modifiés ou non tels que le glycérol, le glycol, le propylène glycol, le dipropylène glycol, le butylène glycol, le butyle diglycol, les silicones volatiles, les huiles minérales, organiques ou végétales, les cires oxyéthylénées ou non, les paraffines, les acides gras, les polymères épaississants associatifs ou non, les amides grasses, les esters gras, les alcools gras, etc.

Les adjuvants cités ci-dessus sont en général présents en quantité comprise, pour chacun d'eux, entre 0,01 et 20% en poids par rapport au poids de la composition.

Il est à noter que si la composition comprend un ou agents épaississants, leur teneur est comprise entre 0,01 et 20% en poids par rapport au poids de la composition lavante, de préférence de 0,01 à 3% en poids par rapport au poids de la composition lavante.

La composition selon l'invention peut contenir un propulseur. Le propulseur est constitué par les gaz comprimés ou liquéfiés usuellement employés pour la préparation de compositions aérosols. On emploiera de manière préférentielle l'air, le gaz carbonique, l'azote comprimé ou encore un gaz soluble tel que le diméthyléther, les hydrocarbures halogénés (fluorés en particuliers) ou non et leurs mélanges.

Les compositions peuvent se présenter sous différentes formes galéniques tels qu'une lotion, un spray, une mousse aérosol, une pompe mousse, etc.

De préférence, la composition lavante (A) comprend au moins un agent conditionneur, de préférence cationique

Selon ce mode de réalisation, la teneur en agent conditionneur dans la composition lavante (A) est comprise entre 0.01 et 20% en poids par rapport au poids de la composition lavante (A).

Selon un mode de réalisation préféré, l'agent conditionneur est un polymère cationique et/ou ou une silicone volatile ou non de préférence une silicone aminée.

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP 337 354 et dans les demandes de brevets français FR 2 270 846, 2 383 660, 2 598 611, 2 470 596 et 2 519 863.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et $5.10^6$ environ, et de préférence comprise entre $10^3$ et $3.\ 10^6$ environ.

Parmi les polymères cationiques, on peut citer plus particulièrement les polymères du type polyamine, polyaminoamide et polyammonium quaternaire.

Les polymères du type polyamine, polyaminoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :

(1) les homopolymères ou copolymères dérivés d'esters ou d'amides acryliques ou méthacryliques et comportant au moins un des motifs de formules suivantes :

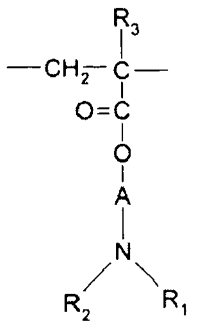 , 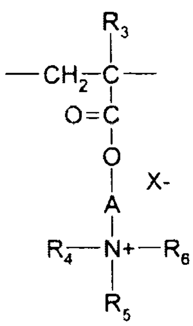

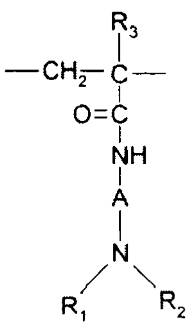 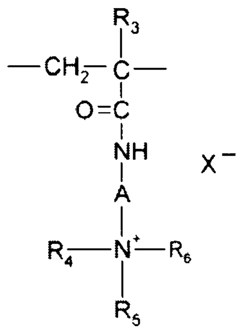

dans lesquelles:

$R_3$, identiques ou différents, désignent un atome d'hydrogène ou un radical $CH_3$ ;
A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
$R_4$, $R_5$, $R_6$, identiques ou différents, représentent un groupe alkyle ayant de 1 à 18 atomes de carbone ou un radical benzyle et de préférence un groupe alkyle ayant de 1 à 6 atomes de carbone; R 1 et R 2 , identiques ou différents, représentent hydrogène ou un groupe alkyle ayant de 1 à 6 atomes de carbone et de préférence méthyle ou éthyle ;
X désigne un anion dérivé d'un acide minéral ou organique tel que un anion méthosulfate ou un halogénure tel que chlorure ou bromure.

Les copolymères de la famille (1) peuvent contenir en outre un ou plusieurs motifs dérivant de comonomères pouvant être choisis dans la famille des acrylamides, méthacrylamides, diacétones acrylamides, acrylamides et mé-thacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.

Ainsi, parmi ces copolymères de la famille (1), on peut citer :

- les copolymères d'acrylamide et de diméthylaminoéthyl méthacrylate quaternisé au sulfate de diméthyle ou avec un halogénure de diméthyle tels que celui vendu sous la dénomination Hercofloc® par la société Hercules,
- les copolymères d'acrylamide et de chlorure de méthacryloyloxyéthyl-triméthylammonium décrit par exemple dans la demande de brevet EP-A-080976 et vendus sous la dénomination BINA QUAT P 100® par la société Ciba Geigy,
- le copolymère d'acrylamide et de méthosulfate de méthacryloyloxyéthyl-triméthylammonium vendu sous la dénomination Reten® par la société Hercules,
- les copolymères vinylpyrrolidone / acrylate ou méthacrylate de dialkylami-noalkyle quaternisés ou non, tels que les produits vendus sous la dénomina- tion "Gafquat" par la société ISP comme par exemple "Gafquat 734" ou "Gafquat 755" ou bien les produits dénommés "Copolymer 845, 958 et 937". Ces polymères sont décrits en détail dans les brevets FR 2077143 et FR 2393573,
- les terpolymères méthacrylate de diméthyl amino éthyle/ vinylcaprolactame/ vinylpyrrolidone tel que le produit vendu sous la dénomination Gaffix® VC 713 par la société ISP,
- les copolymère vinylpyrrolidone / méthacrylamidopropyl dimethylamine commercialisés notamment sous la dénomination Styleze® CC 10 par ISP, et
- les copolymères vinylpyrrolidone / méthacrylamide de diméthylaminopropyle quaternisé tel que le produit vendu sous la dénomination "Gafquat® HS 100" par la société ISP.

(2) Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.

(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl- celluloses greffées notamment avec un sel de méthacryloyléthyl triméthylammonium, de méthacrylamidopropyl triméthylammonium, de diméthyl-diallylammonium.
Les produits commercialisés répondant à cette définition sont plus particulièrement les produits vendus sous la dénomination "CELQUAT ® L 200" et "CELQUAT ® H 100" par la Société National Starch.

(4) Les polysaccharides cationiques décrits plus particulièrement dans les brevets US 3 589 578 et 4 031 307 tels que les gommes de guar contenant des groupements cationiques trialkylammonium. On utilise par exemple des gommes de guar modifiées par un sel (par ex. chlorure) de 2,3-époxypropyl triméthylammonium.
De tels produits sont commercialisés notamment sous les dénominations commerciales de JAGUAR ® C13 S, JAGUAR ® C 15, JAGUAR ® C 17 ou JAGUAR ® C162 par la société RHODIA CHIMIE.

(5) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets FR 2162025 et FR 2280361.

(6) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohy-drine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets FR 2252840 et FR 2368508.

(7) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet FR 1583363.

Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.

(8) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyami- noamide compris entre 0,5 : 1 et 1,8 : 1. De tels polymères sont notamment décrits dans les brevets US 3227615 et US 2961347.
Des polymères de ce type sont en particulier commercialisés sous la dénomination "Hercosett 57" par la société Hercules Inc. ou bien sous la dé- nomination de "PD 170" ou "Delsette 101" par la société Hercules dans le cas du copolymère d'acide adipique/époxypropyl/diéthylène-triamine.

(9) les cyclopolymères d'alkyl diallyl amine ou de dialkyl diallyl ammonium tels que les homopolymères ou copolymères comportant comme constituant principal de la chaîne des motifs répondant aux formules (Va) ou (Vb) :

(Va)
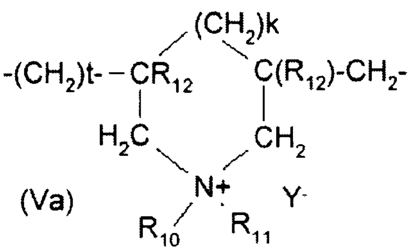

(Vb)
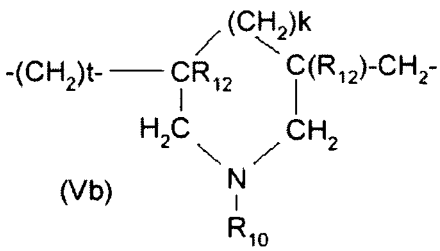

formules dans lesquelles k et t sont égaux à 0 ou 1, la somme k + t étant égale à 1 ; $R_{12}$ désigne un atome d'hydrogène ou un radical méthyle ; $R_{10}$ et $R_{11}$, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C1-C4) ou $R_{10}$ et R11,peuvent désigner conjointement avec l'atome d'azote auquel ils sont rattachés, des groupements hétérocycliques, tels que pipéridinyle ou morpholinyle ; Y - est un anion tel que bromure, chlorure, acétate, borate, citrate, tartrate, bisulfate, bisulfite, sulfate, phosphate. Ces polymères sont notamment décrits dans le brevet FR 2080759 et dans son certificat d'addition 2.190.406.
$R_{10}$ et $R_{11}$, indépendamment l'un de l'autre, désignent de préférence un groupement alkyle ayant de 1 à 4 atomes de carbone.
Parmi les polymères définis ci-dessus, on peut citer plus particulièrement l'homopolymère de chlorure de diméthyldiallylammonium vendu sous la dénomination "Merquat® 100" par la société Nalco (et ses homologues de faibles masses moléculaires moyenne en poids) et les copolymères de chlorure de diallyldiméthylammonium et d'acrylamide commercialisés sous la dénomination "Merquat® 550".

(10) le polymère de diammonium quaternaire contenant des motifs récurrents répondant à la formule (VI) :

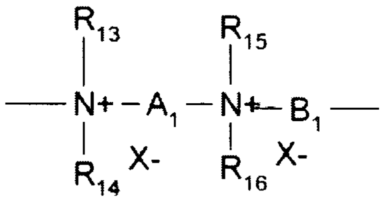 (VI)

formule (VI) dans laquelle :

$R_{13}$, $R_{14}$, $R_{15}$ et $R_{16}$, identiques ou différents, représentent des radicaux aliphatiques, alicycliques, ou arylaliphatiques contenant de 1 à 20 atomes de carbone ou des radicaux hydroxyalkylaliphatiques inférieurs, ou bien $R_{13}$, $R_{14}$, $R_{15}$ et $R_{16}$, ensemble ou séparément, constituent avec les atomes d'azote auxquels ils sont rattachés des hétérocycles contenant éventuellement un second hétéroatome autre que l'azote ou bien $R_{13}$, $R_{14}$, $R_{15}$ et $R_{16}$ représentent un radical alkyle en $C_1$-$C_6$ linéaire ou ramifié substitué par un groupement nitrile, ester, acyle, amide ou -CO-O-$R_{17}$ -D ou -CO-NH-$R_{17}$ - D où $R_{17}$ est un alkylène et D un groupement ammonium quaternaire ;
$A_1$ et $B_1$ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et $X^-$ désigne un anion dérivé d'un acide minéral ou organique, de préférence Cl-, Br ;
$A_1$, $R_{13}$ et $R_{15}$ peuvent former avec les deux atomes d'azote auxquels ils sont rattachés un cycle pipérazinique ; en outre si $A_1$ désigne un radical alkylène ou hydroxyalkylène linéaire ou ramifié, saturé ou insaturé, $B_1$ peut également désigner un groupement $(CH_2)_n$-CO-D-OC-$(CH_2)_n$-
dans lequel D désigne : a) un reste de glycol de formule : -O-Z-O-, où Z désigne un radical hydrocarboné linéaire ou ramifié ou un groupement répondant à l'une des formules suivantes : -($CH_2$-$CH_2$-O)x-$CH_2$-$CH_2$- / -[$CH_2$-CH ($CH_3$)-O$]_y$-$CH_2$-CH($CH_3$)- où x et y désignent un nombre entier de 1 à 4, représentant un degré de polymérisation défini et unique ou un nombre quelconque de 1 à 4 représentant un degré de polymérisation moyen ; b) un reste de diamine bis-secondaire tel qu'un dérivé de pipérazine ; c) un reste de diamine bis-primaire de formule : -NH-Y-NH-, où Y désigne un radical hydrocarboné linéaire ou ramifié, ou bien le radical bivalent -$CH_2$-$CH_2$-S-S-$CH_2$-$CH_2$- ; d) un groupement uréylène de formule : -NH-CO-NH- ; n varie de 1 à 6.
Ces polymères ont une masse moléculaire moyenne en nombre généralement comprise entre 1000 et 100000.
Des polymères de ce type sont notamment décrits dans les brevets FR 2320330, FR 2270846, FR 2316271, FR 2336434 et FR 2413907 et les brevets US 2273780, US 2375853, US 2388614, US 2454547, US 3206462, US 2261002, US 2271378, US 3874870, US 4001432, US 3929990, US 3966904, US 4005193, US 4025617, US 4025627, US 4025653, US 4026945 et US 4027020.
On peut utiliser plus particulièrement les polymères qui sont constitués de motifs récurrents répondant à la formule :

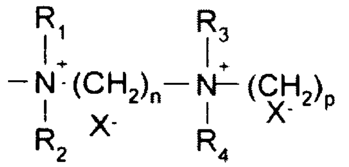 (VII)

dans laquelle $R_1$, $R_2$, $R_3$ et $R_4$, identiques ou différents, désignent un radical alkyle ou hydroxyalkyle ayant de 1 à 4 atomes de carbone environ, n et p sont des nombres entiers variant de 2 à 20 environ et, X - est un anion dérivé d'un acide minéral ou organique.
Un composé de formule (VII) particulièrement préféré est celui pour lequel $R_1$, $R_2$, $R_3$ et $R_4$, représentent un radical méthyle et n = 3, p = 6 et X = Cl, dénommé Hexadimethrine chloride selon la nomenclature INCI (CTFA).

(11) les polymères de polyammonium quaternaires constitués de motifs de formule (VIII):

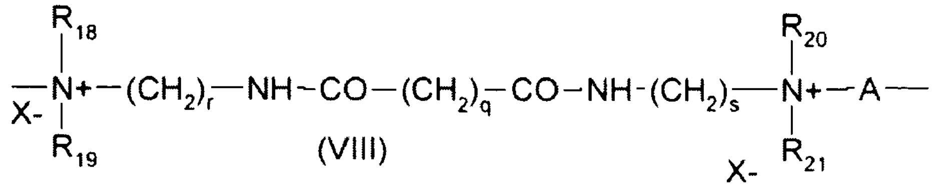

(VIII)

formule dans laquelle :

$R_{18}$, $R_{19}$, $R_{20}$ et $R_{21}$ , identiques ou différents, représentent un atome d'hydrogène ou un radical méthyle, éthyle, propyle, ®-hydroxyéthyle, ® - hydroxypropyle ou $-CH_2CH_2(OCH_2CH_2)_pOH$, où p est égal à 0 ou à un nombre entier compris entre 1 et 6, sous réserve que $R_{18}$, $R_{19}$, $R_{20}$ et $R_{21}$ ne représentent pas simultanément un atome d'hydrogène, r et s, identiques ou différents, sont des nombres entiers compris entre 1 et 6, q est égal à 0 ou à un nombre entier compris entre 1 et 34, X- désigne un anion tel qu'un halogénure, A désigne un radical d'un dihalogénure ou représente de préférence $-CH_2-CH_2-O-CH_2-CH_2-$.

De tels composés sont notamment décrits dans la demande de brevet EP 122 324.

On peut par exemple citer parmi ceux-ci, les produits "Mirapol® A 15", "Mirapol® AD1", "Mirapol® AZ1" et "Mirapol® 175" vendus par la société Miranol.

(12) Les polymères quaternaires de vinylpyrrolidone et de vinylimidazole tels que par exemple les produits commercialisés sous les dénominations Luvi-quat® FC 905, FC 550 et FC 370 par la société BASF.

(13) Les polyamines comme le Polyquart® H vendu par COGNIS, référencé sous le nom de « POLYETHYLENEGLYCOL (15) TALLOW POLYAMINE » dans le dictionnaire CTFA.

(14) Les polymères réticulés de sels de méthacryloyloxyalkyl($C_1$-$C_4$) trialkyl($C_{1}$-$C_4$)ammonium tels que les polymères obtenus par homopolymérisation du diméthylaminoéthylméthacrylate quaternisé par le chlorure de méthyle, ou par copolymérisation de l'acrylamide avec le diméthylaminoéthylmé-thacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl tri-méthylammonium (20/80 en poids) sous forme de dispersion contenant 50 % en poids dudit copolymère dans de l'huile minérale. Cette dispersion est commercialisée sous le nom de Salcare ® SC 92 par la société Ciba Geigy. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de Salcare ® SC 95 et Salcare ® SC 96 par la société Ciba Geigy.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les dérivés d'éther de cellulose quaternaires tels que les produits vendus sous la dénomination JR 400 par la société Amerchol, les cyclopolymères cationiques, en particulier les homopolymères ou copolymères de chlorure de diméthyldiallylammonium, vendus sous les dénominations Merquat® 100, Merquat® 550 et Merquat® S par la société Nalco, les polysaccharides cationiques tels que les gommes de guar modifiées par un sel de 2,3-époxypropyl triméthylammonium, les polymères quaternaires de vinylpyr-rolidone et de vinylimidazole et leurs mélanges.

Lors d'une étape c), qui peut être mise en oeuvre immédiatement ou non après l'étape b), ou bien après l'étape a), les fibres kératiniques sont traitées avec une composition lavante (B). De préférence, lorsque l'étape c) a lieu après l'étape b), ce traitement a lieu au moins 12 heures après l'application de la composition colorante, de préférence au moins 24 heures après.

La composition lavante (B) comprend à titre de tensioactif, au moins un tensioactif amphotère et/ou au moins un tensioactif zwittérionique et/ou au moins un tensioactif doux et/ou au moins un tensioactif non ionique choisi parmi les alkylpolyglucosides, les tensioactifs monoglycérolés ou polyglycérolés.

Ce qui a été indiqué précédemment au sujet de la nature des tensioactifs amphotères, zwittérioniques et anioniques doux reste valable et ne sera pas repris à nouveau.

En ce qui concerne les alkypolyglucosides, ces composés sont bien connus et peuvent être plus particulièrement

représenté par la formule générale suivante :

$$R_1O\text{-}(R_2O)_t\ (G)_v$$

dans laquelle $R_1$ représente un radical alkyle et/ou alcényle linéaire ou ramifié comportant environ de 8 à 24 atomes de carbone, un radical alkylphényle dont le radical alkyle linéaire ou ramifié comporte de 8 à 24 atomes de carbone, $R_2$ représente un radical alkylène comportant environ de 2 à 4 atomes de carbone, G représente un sucre réduit comportant de 5 à 6 atomes de carbone, t désigne une valeur allant de 0 à 10, de préférence 0 à 4, de préférence 0 à 4 et v désigne une valeur allant de 1 à 15.

On peut utiliser par exemple, l'Alkyl en C8/C16 polyglucoside 1.4 en solution aqueuse à 53% commercialisé par Cognis sous la référence Plantacare® 818 UP.

Pour ce qui a trait aux tensioactifs monoglycérolés ou polyglycérolés, la composition lavante comprend de préférence au moins un composé de formules suivantes : $RO[CH_2CH(CH_20H)O]_mH$ ou $RO[CH(CH_20H)CH_20]_mH$ ou $RO[CH_2CH(OH)CH_20]_mH$ ; dans lesquelles R représente un radical saturé ou insaturé, linéaire ou ramifié, comportant de 8 à 40 atomes de carbone et de préférence de 10 à 30 atomes de carbone ; m est un nombre compris entre 1 et 30, de préférence entre 1 et 10, plus particulièrement de 1,5 à 6.

On peut utiliser par exemple, l'alcool dodécanediol polyglycérolé (3.5 moles) commercialisé sous la dénomination Chimexane® NF de Chimex.

Conformément à un mode de réalisation particulier de l'invention, la teneur totale en tensioactif amphotère, zwittérionique, anionique doux et/ou non ionique est comprise entre 4 et 50 % en poids par rapport au poids de la composition lavante (B), de préférence entre 6 et 30 % en poids par rapport au poids de la composition lavante (B).

Il est à noter que la composition lavante (B) peut comprendre au moins un tensioactif détergent anionique, et plus particulièrement les alkyléthersulfates, les alkyl sulfates.

Si de tels tensioactifs sont présents, alors la teneur totale en tensioactif(s) détergent(s) de ce type est comprise entre 4 et 50% en poids, par rapport à la teneur totale en tensioactifs détergents présents dans la composition lavante (B), plus particulièrement compris entre 6% et 30 % en poids, par rapport à la même référence.

De préférence, la composition lavante (B) ne contient pas de tensioactif détergent anionique de type alkylsulfate ou alkylether sulfate. Et si elle en contient, sa teneur est telle que le rapport pondéré : tensioactif détergent anionique de type alkylsulfate ou alkylether sulfate / somme des tensioactifsamphotère, zwittérionique, anionique doux et/ou non ionique soit inférieur ou égal à 0,6, et plus particulièrement inférieur ou égal à 0,2.

Selon un mode de réalisation préféré de l'invention, la composition lavante (B) comprend au moins un agent conditionneur, de préférence cationique.
On pourra se reporter à la description de ces composés donnée dans le cadre de la description de la deuxième composition lavante (B).

Selon ce mode de réalisation préféré, la teneur en agent conditionneur dans la deuxième composition lavante est comprise entre 0.01 et 20% en poids par rapport au poids de la première composition lavante.

Selon un autre mode de réalisation préféré de l'invention, la composition lavante (B) comprend au moins un agent épaississant identique à ceux décrits pour la composition lavante (A).

Lorsqu'un agent épaississant est employé, sa teneur dans la deuxième composition lavante (B) est comprise entre 0,01 et 20 % en poids par rapport au poids, de la composition lavante (B).

Enfin, la composition lavante (B) peut comprendre les additifs classiques dans le domaine, et notamment ceux mentionnés dans le cadre de la composition lavante (A), dans les teneurs habituelles indiquées auparavant.

Une fois le ou les lavages réalisés, on sèche les fibres ou on les laisse sécher.

Conformément à une première variante de l'invention, l'étape c) du procédé, c'est-à-dire le lavage mettant en oeuvre la composition lavante (B), est réalisée directement à l'issue de l'étape a) du procédé (étape de coloration).

Ainsi, les fibres kératiniques sont rincées pour éliminer l'excès de composition colorante puis ensuite lavées une ou plusieurs fois avec la composition lavante (B), avec habituellement un rinçage intermédiaire.

A noter que l'étape c) peut être mise en oeuvre plusieurs fois, avec la fréquence classiquement utilisée entre deux shampooings (c'est-à-dire par exemple avec des intervalles de temps compris entre 12 heures et 2 semaines).

Selon une autre possibilité, on met en oeuvre l'étape c) de lavage avec la composition lavante (B), de manière différée par rapport à une étape b) antérieure et/ou par rapport à une étape c) antérieure.

Dans ce cas, le procédé consiste à mettre en oeuvre successivement, les étapes a) et b), ou a) et c), puis à l'issue de la seconde étape (soit l'étape b) soit l'étape c)) à sécher ou à laisser les fibres. L'étape c) est ensuite mise en oeuvre après un temps plus ou moins long, de préférence au moins 12 heures après l'application, ou encore de préférence au moins 24 heures après l'application de la composition colorante.

Un autre objet de la présente invention est constitué par un kit pour la mise en oeuvre du procédé selon l'invention comprenant tout d'abord une composition colorante et éventuellement une composition comprenant au moins un agent oxydant ; puis éventuellement au moins une composition lavante (A) et enfin au moins une composition lavante

(B).

Tout ce qui a été précédemment détaillé concernant la nature des éléments constitutifs des diverses compositions ainsi que leurs proportions, reste valable et ne sera pas repris dans cette partie de la description.

Enfin, un autre objet de l'invention est constitué par l'utilisation d'une composition lavante comprenant, à titre de tensioactif, au moins un tensioactif amphotère et/ou au moins un tensioactif zwittérionique et/ou au moins un tensioactif anionique doux et/ou au moins un tensioactif non ionique exclusivement choisi parmi les alkylpolyglycosides, les tensioactifs monoglycérolés ou polyglycérolés, ou leurs mélanges, pour la protection de la coloration et/ou pour la limitation du dégorgement de la coloration, de fibres kératiniques, coloration obtenue au moyen d'une composition de coloration comprenant au moins une paraphénylènediamine cationique telle que décrite auparavant.

De préférence, la teneur totale en tensioactifs amphotères, zwittérioniques, anioniques doux et non ioniques est comprise entre 4 et 50 % en poids par rapport au poids de la composition lavante, de préférence entre 6 et 30 % en poids par rapport au poids de la composition lavante.

En outre, cette composition lavante peut comprendre au moins un tensioactif détergent anionique additionnel de préférence choisi parmi les alkyléthers sulfate et/ou les alkyl sulfate.

De préférence, la composition lavante ne contient pas de tensioactif détergent anionique, de type alkylsulfate ou alkylether sulfate. Et si elle en contient, sa teneur est telle que le rapport pondéré : tensioactif détergent anionique de type alkylsulfate ou alkylether sulfate / somme des tensioactifsamphotère, zwittérionique, anionique doux et/ou non ionique soit inférieur ou égal à 0,6, et plus particulièrement inférieur ou égal à 0,2.

Il est noter que la composition lavante peut comprendre au moins un agent conditionneur, de préférence cationique.

Par ailleurs, au cas où la composition comprend un tel agent, sa teneur est comprise entre 0.01 et 20 % en poids par rapport au poids de la composition lavante.

Il est rappelé que l'on pourra se reporter à la description, pour ce qui est de la nature plus précise des ingrédients compris dans cette composition lavante.

Des exemples concrets mais non limitatifs de l'invention vont maintenant être présentés.

**<u>EXEMPLES</u>**

Les compositions de teinture suivantes ont été réalisés à partir des différentes bases d'oxydation décrites ci-dessous :

| | |
|---|---|
| Paraphénylène diamine | 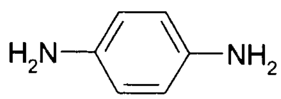 |
| Chlorure de 1-[1-(4-aminophenyl)pyrrolidin-3-yl]-3-methyl-1H-imidazol-3-ium, monochlorhydrate | 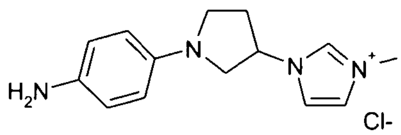 , HCl |
| Dichlorure de propane-1,3-bis-(N-(3-Imidazolium-N-propyl)-benzene-1,4-diamine, tétrachlorhydrate | 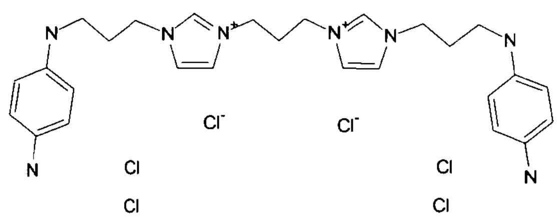 |

Tableau 1

| | Composition 1 | Composition 2 | Composition 3 |
|---|---|---|---|
| Décyl glucoside en solution aqueuse à 60% d'Oramix® CG 110 (SEPPIC) | 5,4g | 5,4g | 5,4g |
| Alcool éthylique 96 degrés dénaturé | 18g | 18g | 18g |
| Alcool benzylique | 1,8g | 1,8g | 1,8g |
| Polyéthylène glycol (8 OE) | 2,7g | 2,7g | 2,7g |
| Pentasodium pentetate à 40% dans l'eau | 1,08g | 1,08g | 1,08g |
| Sodium Métabisulfite | 0,205g | 0,205g | 0,205g |
| paraphénylène diamine | $6.10^{-3}$ mol | - | - |
| 1-[1-(4-aminophenyl)pyrrolidin-3-yl]-3-methyl-1 H-imidazol-3-ium | - | $6.10^{-3}$ mol% | - |
| Propane-1,3-bis-(N-(3-Imidazolium-N-propyl)-benzene-1,4-diamine | - | - | $3.10^{-3}$ mol% |
| 1-méthyl-4-amino-phénol | $6.10^{-3}$ mol | $6.10^{-3}$ mol | $6.10^{-3}$ mol |
| Ammoniaque à 20.5% | 10g | 10g | 10g |
| Eau déminéralisée | Qsp 100 | Qsp 100 | Qsp 100 |

Au moment de l'emploi, chacune des compositions ci-dessus est mélangée avec de l'eau oxygénée (Eau oxygénée l'Oréal professionnel 20 volumes à 6%) poids pour poids.

Le mélange est ensuite appliqué sur des mèches de cheveux naturels à 90% blancs fortement décolorées et permanentées, à raison de 10 g de mélange colorant/ g de mèche. Le temps de pause est de 15 minutes de chaque côté de la mèche.

La coloration est ensuite rincée à l'eau puis lavée avec un shampooing commercial (shampooing DOP camomille). Les mèches sont ensuite séchées 30 minutes à 60°C au casque.

Etapes de lavage :

48 heures après la réalisation des colorations précédemment décrites, les mèches subissent des épreuves de lavages par un shampooing contenant un tensioactif non ionique (composition détergente 1 du tableau 2)

Tableau 2

| | Composition détergente 1 |
|---|---|
| Coco-glucoside à 53% dans l'eau (Plantacare® 818 UP-Cognis) | 28,30g |
| Acide citrique | qsp pH=6 |
| Eau | qsp 100 |

On réalise ainsi 10 shampooings, les mèches étant séchées au casque (30 minutes à 60°C) entre chaque shampooing.

Avant et après les 10 étapes de lavage, la coloration des mèches est évaluée visuellement et par la mesure des valeurs (L*, a*, b*) à l'aide du spectro-colorimètre MINOLTA CM 2022.

La dégradation de la coloration avant et après les 10 étapes de lavage au moyen de la composition détergente 1 est exprimée en ΔE selon la formule suivante :

$$\Delta E_{i(\text{après lavage - avant lavage})} = \sqrt{(\Delta L_i^{*2} + \Delta a_i^{*2} + \Delta b_i^{*2})}$$

Les résultats après 10 étapes de lavages sont rassemblés dans le tableau 3 suivant :

Tableau 3

| Coloration | Composition 1 (comparatif) | Composition 2 (inv) | Composition 3 (inv) |
|---|---|---|---|
| Dégradation de la coloration (□E) | 23,2 | 6,8 | 0,6 |

Ces résultats montrent une très nette amélioration de la tenue de la coloration lorsque l'on applique la composition détergente 1 sur des mèches colorées par des compositions contenant une base d'oxydation para-phénylènediamine cationique.

Il est également constaté que le dégorgement de la couleur dans l'eau et dans la mousse est moins important dans le cas de la présente invention.

## Revendications

**1.** Procédé de traitement de fibres kératiniques humaines dans lequel on met en oeuvre les étapes suivantes :

a) on réalise une étape de coloration desdites fibres au moyen d'une composition prête à l'emploi à base d'une composition colorante comportant au moins une base d'oxydation du type paraphénylènediamine substituée par au moins un radical cationique ;
b) éventuellement, on lave les fibres avec une composition lavante (A) comprenant au moins un tensioactif détergent anionique choisi parmi les alkyléthers sulfate et/ou les alkyl sulfate, on les rince et éventuellement on les sèche ou on les laisse sécher ;
c) on lave les fibres avec une composition lavante (B) comprenant au moins un tensioactif amphotère et/ou au moins un tensioactif zwittérionique et/ou au moins un tensioactif anionique doux et/ou au moins un tensioactif non ionique choisi parmi les alkylpolyglucosides, les tensioactifs monoglycérolés ou polyglycérolés, puis on rince les fibres et on les sèche ou on les laisse sécher.

**2.** Procédé selon la revendication 1 dans lequel le radical cationique est un radical ammonium quaternaire ou un radical comportant un atome d'azote quaternisé inclus dans un cycle.

**3.** Procédé selon la revendication 2 dans lequel le radical cationique est choisi parmi les radicaux diéthyl-méthyl-ammonium, triméthylammonium, diéthyl-(2-hydroxyéthyl)-ammonium, 1,4-diméthyl-pipérazin-1-ium, pyrrolium, imidazolium, pyrazolium, oxazolium, thiazolium, triazolium, pyridinium, pyrimidinium, pyrazinium, oxazinium et triazinium.

**4.** Procédé selon la revendication 4 dans lequel le radical cationique est un radical imidazolium ou pyridinium.

**5.** Procédé selon l'une quelconque des revendications précédentes dans lequel la base d'oxydation est une base double para-phénylènediamine.

**6.** Procédé selon l'une quelconque des revendications 1 à 4 dans lequel la base d'oxydation est une paraphénylènediamine à groupement pyrrolidinyle substituée par un ou plusieurs radicaux cationiques.

**7.** Procédé selon l'une quelconque des revendications précédentes dans lequel la ou les bases d'oxydation sont présentes chacune en quantité comprise entre 0,001 et 10% en poids par rapport au poids de la composition de coloration.

**8.** Procédé selon l'une quelconque des revendications précédentes dans lequel la composition de coloration d'oxydation contient un coupleur.

**9.** Procédé selon la revendication 8 dans lequel le coupleur est un coupleur méta-phénylènediamine dont les deux groupements amino sont des groupement amino primaires.

**10.** Procédé selon la revendication 8 dans lequel le ou les coupleurs méta-phénylènediamine sont choisis parmi la métaphénylène diamine, le 2,4-diamino phénoxyéthanol, le 1-méthoxy 2-amino 4-(2'-hydroxy éthylamino)benzène, la 4-fluoro-6-methylbenzene-1,3-diamine, le 1-amino 3-NN bis β hydroxyéthylaminobenzène, le 1,3 bis (2,4-diaminophenoxy)propane, le 1-β-aminoéthyloxy-2,4-diaminobenzène, le 1-β-hydroxyéthyl-2,4-diaminobenzène, l'acide (2,4-diaminophénoxy)acétique, le 4,6-bis (β-hydroxyéthoxy)-1,3-diaminobenzène, le 2,4-diamino 5-méthyléthoxybenzène, le 2,4-diamino 5-β-hydroxyéthyloxytoluène, le 2,4-diméthoxy 1,3-diaminobenzène ou leurs sels d'addition.

**11.** Procédé selon la revendication 8 dans lequel le ou les coupleurs sont présents chacun en quantité comprise entre 0,001 et 10% en poids par rapport au poids de la composition de coloration.

**12.** Procédé selon l'une quelconque des revendications précédentes dans lequel la composition de coloration d'oxydation comprend au moins un agent oxydant.

**13.** Procédé selon la revendication précédente, **caractérisée en ce que** l'agent oxydant est choisi parmi le peroxyde d'hydrogène, le peroxyde d'urée, les bromates ou ferricyanures de métaux alcalins, les persels, les enzymes d'oxydoréduction telles que les laccases, les peroxydases et les oxydoréductases à 2 électrons, le cas échéant en présence de leur donneur respectif.

**14.** Procédé selon l'une des revendications précédentes, **caractérisé en ce que** la composition prête à l'emploi est obtenue par mélange avant l'application sur les fibres, d'une composition colorante avec une composition comprenant au moins un agent oxydant.

**15.** Procédé selon l'une quelconque des revendications précédentes, **caractérisé en ce que** la teneur en tensioactif détergent anionique dans la composition lavante

(A) est comprise entre 4 à 50% en poids par rapport au poids de la première composition lavante et de préférence de 6 à 30% en poids par rapport au poids de la composition lavante (A).

**16.** Procédé selon l'une quelconque des revendications précédentes, **caractérisé en ce que** la composition lavante (B) comprend au moins un alkylpolyglucoside représenté par la formule générale suivante :

$$R_1O\text{-}(R_2O)_t\,(G)_v$$

dans laquelle $R_1$ représente un radical alkyle et/ou alcényle linéaire ou ramifié comportant environ de 8 à 24 atomes de carbone, un radical alkylphényle dont le radical alkyle linéaire ou ramifié comporte de 8 à 24 atomes de carbone, $R_2$ représente un radical alkylène comportant environ de 2 à 4 atomes de carbone, G représente un sucre réduit comportant de 5 à 6 atomes de carbone, t désigne une valeur allant de 0 à 10, de préférence 0 à 4, de préférence 0 à 4, de préférence 0 à 4, et v désigne une valeur allant de 1 à 15.

**17.** Procédé selon l'une quelconque des revendications précédentes, **caractérisé en ce que** la composition lavante (B) comprend au moins un tensioactif monoglycérolé ou polyglycérolé de formules suivantes $RO[CH_2CH(CH_2OH)O]_mH$ ou $RO[CH(CH_2OH)CH_2O]_mH$ ou $RO[CH_2CH(OH)CH_2O]_mH$ ; dans lesquelles R représente un radical saturé ou insaturé, linéaire ou ramifié, comportant de 8 à 40 atomes de carbone et de préférence de 10 à 30 atomes de carbone ; m est un nombre compris entre 1 et 30, de préférence entre 1 et 10, plus particulièrement de 1,5à6.

**18.** Procédé selon l'une quelconque des revendications précédentes, **caractérisé en ce que** la composition lavante (B) comprend, à titre de tensioactif amphotère ou zwittérionique, au moins un composé choisi parmi les dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant ; les alkyl ($C_8$-$C_{20}$) bétaïnes, les sulfobétaïnes, les alkyl ($C_8$-$C_{20}$) amidoalkyl ($C_1$-$C_6$) bétaïnes ou les alkyl ($C_8$-$C_{20}$) amidoalkyl ($C_1$-$C_6$) sulfobétaïnes, seuls ou en mélange.

**19.** Procédé selon l'une quelconque des revendications précédentes, **caractérisé en ce que** la composition lavante (B) comprend au moins un tensioactif anionique doux choisi parmi, seul ou en mélange :

- les acides alkyl éther carboxyliques polyoxyalkylénés,
- les acides alkylaryl éther carboxyliques polyoxyalkylénés,
- les acides alkylamido éther carboxyliques polyoxyalkylénés en particulier ceux comportant 2 à 50 groupements oxyde d'éthylène,
- les acides d'alkyl D galactoside uroniques
- les acylsarcosinates, les acylglutamates ;

- les esters d'alkylpolyglycosides carboxyliques ;
- les sels d'acides gras.

**20.** Procédé selon la revendication précédente, **caractérisé en ce que** la teneur totale en tensioactif amphotère, zwittérionique, anionique doux et/ou non ionique est comprise entre 4 et 50 % en poids par rapport au poids de la composition lavante (B), de préférence entre 6 et 30 % en poids par rapport au poids de la composition lavante (B).

**21.** Procédé selon l'une quelconque des revendications précédentes, **caractérisée en ce que** la composition lavante (A) et/ou la composition lavante (B) comprend au moins un agent conditionneur, de préférence cationique.

**22.** Procédé selon la revendication précédente, **caractérisé en ce que** l'agent conditionneur est un polymère cationique ou une silicone.

**23.** Procédé selon la revendication précédente, **caractérisé en ce que** l'agent conditionneur est une silicone aminée.

**24.** Procédé selon l'une des revendications 21 à 23, **caractérisé en ce que** la teneur en agent conditionneur dans les compositions lavantes (A) et/ou (B) est comprise entre 0,01 et 20 % en poids par rapport au poids, respectivement des compositions lavantes (A) et/ou (B).

**25.** Procédé selon l'une quelconque des revendications précédentes, **caractérisée en ce que** les compositions lavantes (A) et/ou (B) comprend au moins un agent épaississant.

**26.** Procédé selon la revendication précédente, **caractérisée en ce que** la teneur en agent épaississant dans les compositions lavantes (A) et/ou (B) est comprise entre 0,01 et 20% en poids par rapport au poids, respectivement des compositions lavantes (A) ou (B).

**27.** Procédé selon l'une quelconque des revendication précédente, **caractérisé en ce que** la composition lavante (B) est conditionnée dans un dispositif aérosol.

**28.** Procédé selon la revendication précédente, **caractérisé en ce que** le dispositif contient un propulseur.

**29.** Procédé selon la revendication précédente, **caractérisé en ce que** le propulseur est choisi parmi les gaz comprimés ou liquéfiés.

**30.** Procédé selon l'une quelconque des revendications précédentes, **caractérisé en ce que** l'étape c) du procédé est réalisée directement à l'issue de l'étape a) du procédé.

**31.** Procédé selon la revendication précédente, **caractérisé en ce que** l'on rince les fibres kératiniques pour éliminer l'excès de composition colorante puis on lave lesdites fibres une ou plusieurs fois avec la composition lavante (B), avec habituellement un rinçage intermédiaire.

**32.** Procédé selon l'une quelconque des revendications précédentes, **caractérisé en ce que** l'application de la composition lavante (B) s'effectue au moins 12 heures après l'application de la composition colorante, de préférence au moins 24 heures après l'application.

**33.** Procédé selon l'une des revendications précédentes, **caractérisé en ce que** l'étape c) est mise en oeuvre plusieurs fois, avec la fréquence classiquement utilisée entre deux shampooings.

**34.** Procédé selon la revendication précédente, **caractérisé en ce que** la fréquence est comprise entre 12 heures et 2 semaines.

**35.** Procédé selon l'une quelconque des revendications 1 à 29, **caractérisé en ce que** l'on met en oeuvre l'étape c), de manière différée par rapport à une étape b) antérieure et/ou par rapport à une étape c) antérieure.

**36.** Procédé selon la revendication précédente, **caractérisé en ce que** l'on met en oeuvre successivement, les étapes a) et b), ou a) et c), puis à l'issue de la seconde étape, soit l'étape b) soit l'étape c), à sécher ou à laisser les fibres ; puis on met en oeuvre l'étape c).

**37.** Procédé selon la revendication précédente, **caractérisé en ce que** l'étape c)est mise en oeuvre au moins 12 heures après l'application, ou encore de préférence au moins 24 heures après l'application de la composition colorante.

**38.** Kit pour la mise en oeuvre du procédé selon l'une quelconque des revendications précédentes, **caractérisé en ce qu'**il comprend une composition colorante et éventuellement une composition comprenant au moins un agent oxydant ; éventuellement au moins une composition lavante (A), et au moins une composition lavante (B).

**39.** Utilisation d'une composition lavante comprenant, à titre de tensioactif, au moins un tensioactif amphotère, au moins un tensioactif zwittérionique, au moins un tensioactif anionique doux et/ou ou au moins un tensioactif non ionique exclusivement choisi parmi les alkylpolyglycosides, les tensioactifs monoglycérolés ou polyglycérolés, ou leurs mélanges, pour la limitation du dégorgement et/ou l'amélioration de la protection de la coloration de fibres kératiniques, en relation avec l'utilisation préalable d'une composition de coloration comprenant au moins une base d'oxydation du type paraphénylènediamine substituée par au moins un radical cationique.

**40.** Utilisation selon la revendication précédente, **caractérisée en ce que** la teneur totale en tensioactif amphotère, zwittérionique, anionique doux et/ou non ionique est comprise entre 4 et 50 % en poids par rapport au poids de la composition lavante, de préférence entre 6 et 30 % en poids par rapport au poids de la composition lavante.

**41.** Utilisation selon l'une quelconque des revendications 39 ou 40, **caractérisée en ce que** la composition lavante comprend au moins un agent conditionneur, de préférence cationique.

**42.** Utilisation selon la revendications précédente, **caractérisée en ce** l'agent conditionneur est un polymère cationique et/ou une silicone aminée.

**43.** Utilisation selon la revendication précédente, **caractérisé en ce que** la teneur en agent conditionneur est comprise entre 0,01 et 20% en poids par rapport au poids de la composition lavante.

Office européen des brevets

# RAPPORT DE RECHERCHE EUROPEENNE

Numéro de la demande
EP 05 29 2741

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | WO 99/03834 A (L'OREAL; GENET, ALAIN; LAGRANGE, ALAIN) 28 janvier 1999 (1999-01-28) * page 2 - page 3 * & EP 0 932 602 A 4 août 1999 (1999-08-04) cité dans la demande | 1-43 | INV. A61K8/60 A61K8/39 A61K8/46 A61Q5/02 A61Q5/10 |
| D,X | EP 1 348 695 A (L'OREAL) 1 octobre 2003 (2003-10-01) * alinéas [0006], [0014], [0015] * | 1-43 | |
| X | WO 99/03819 A (L'OREAL; GENET, ALAIN; LAGRANGE, ALAIN) 28 janvier 1999 (1999-01-28) * page 8, ligne 18 - ligne 22 * & EP 0 968 171 A 5 janvier 2000 (2000-01-05) cité dans la demande | 1-43 | |
| X | WO 99/03836 A (L'OREAL; GENET, ALAIN; LAGRANGE, ALAIN) 28 janvier 1999 (1999-01-28) * page 10, ligne 9 - ligne 16 * & EP 0 928 289 A 14 juillet 1999 (1999-07-14) cité dans la demande | 1-43 | DOMAINES TECHNIQUES RECHERCHES (IPC) |
| | | | A61K |
| D,X | EP 1 396 486 A (L'OREAL) 10 mars 2004 (2004-03-10) * alinéas [0005], [0015], [0016] * | 1-43 | |
| Y | WO 01/97759 A (HENKEL KOMMANDITGESELLSCHAFT AUF AKTIEN; KLEEN, ASTRID; HOEFFKES, HORS) 27 décembre 2001 (2001-12-27) * exemples 2.7-2.10 * * page 32 * * page 1 - page 2 * | 1-43 | |
| | -/-- | | |

Le présent rapport a été établi pour toutes les revendications

| Lieu de la recherche | Date d'achèvement de la recherche | Examinateur |
|---|---|---|
| Munich | 17 mars 2006 | Simon, F |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

5
EPO FORM 1503 03.82 (P04C02)

Office européen des brevets

# RAPPORT DE RECHERCHE EUROPEENNE

Numéro de la demande
EP 05 29 2741

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | WO 95/22312 A (HENKEL KOMMANDITGESELLSCHAFT AUF AKTIEN; MUELLER, REINHARD; SEIDEL, KU) 24 août 1995 (1995-08-24) * page 2 * | 1-43 | |
| Y | EP 1 321 131 A (HENKEL KOMMANDITGESELLSCHAFT AUF AKTIEN) 25 juin 2003 (2003-06-25) * exemple 8 * * alinéas [0003], [0008] * | 1-43 | |
| Y | EP 1 312 346 A (L'OREAL) 21 mai 2003 (2003-05-21) * alinéas [0001], [0004] * * page 6; exemples D,E * | 1-43 | |
| Y | EP 1 312 345 A (L'OREAL) 21 mai 2003 (2003-05-21) * alinéas [0001], [0003], [0004] * | 1-43 | DOMAINES TECHNIQUES RECHERCHES (IPC) |
| Y | FR 2 705 888 A (OREAL) 9 décembre 1994 (1994-12-09) * page 1 - page 2 * | 1-43 | |

Le présent rapport a été établi pour toutes les revendications

| Lieu de la recherche | Date d'achèvement de la recherche | Examinateur |
|---|---|---|
| Munich | 17 mars 2006 | Simon, F |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
&: membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 05 29 2741

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

17-03-2006

| Document brevet cité au rapport de recherche | | Date de publication | Membre(s) de la famille de brevet(s) | | | Date de publication |
|---|---|---|---|---|---|---|
| WO 9903834 | A | 28-01-1999 | AT | 260900 | T | 15-03-2004 |
| | | | AU | 8735698 | A | 10-02-1999 |
| | | | CA | 2265489 | A1 | 28-01-1999 |
| | | | DE | 69822119 | D1 | 08-04-2004 |
| | | | DE | 69822119 | T2 | 05-01-2005 |
| | | | EP | 0932602 | A2 | 04-08-1999 |
| | | | ES | 2217571 | T3 | 01-11-2004 |
| | | | FR | 2766179 | A1 | 22-01-1999 |
| | | | JP | 2000504351 | T | 11-04-2000 |
| | | | US | 6270533 | B1 | 07-08-2001 |
| EP 0932602 | A | 04-08-1999 | AT | 260900 | T | 15-03-2004 |
| | | | AU | 8735698 | A | 10-02-1999 |
| | | | CA | 2265489 | A1 | 28-01-1999 |
| | | | DE | 69822119 | D1 | 08-04-2004 |
| | | | DE | 69822119 | T2 | 05-01-2005 |
| | | | ES | 2217571 | T3 | 01-11-2004 |
| | | | FR | 2766179 | A1 | 22-01-1999 |
| | | | WO | 9903834 | A2 | 28-01-1999 |
| | | | JP | 2000504351 | T | 11-04-2000 |
| | | | US | 6270533 | B1 | 07-08-2001 |
| EP 1348695 | A | 01-10-2003 | FR | 2837821 | A1 | 03-10-2003 |
| | | | JP | 2003286256 | A | 10-10-2003 |
| WO 9903819 | A | 28-01-1999 | AT | 251607 | T | 15-10-2003 |
| | | | AU | 8735498 | A | 10-02-1999 |
| | | | CA | 2265544 | A1 | 28-01-1999 |
| | | | DE | 69818807 | D1 | 13-11-2003 |
| | | | DE | 69818807 | T2 | 05-08-2004 |
| | | | EP | 0968171 | A1 | 05-01-2000 |
| | | | ES | 2209180 | T3 | 16-06-2004 |
| | | | FR | 2766177 | A1 | 22-01-1999 |
| | | | JP | 2000503036 | T | 14-03-2000 |
| | | | JP | 3379966 | B2 | 24-02-2003 |
| | | | JP | 2002053534 | A | 19-02-2002 |
| | | | JP | 2006022113 | A | 26-01-2006 |
| | | | US | 6565614 | B1 | 20-05-2003 |
| EP 0968171 | A | 05-01-2000 | AT | 251607 | T | 15-10-2003 |
| | | | AU | 8735498 | A | 10-02-1999 |
| | | | CA | 2265544 | A1 | 28-01-1999 |
| | | | DE | 69818807 | D1 | 13-11-2003 |
| | | | DE | 69818807 | T2 | 05-08-2004 |
| | | | ES | 2209180 | T3 | 16-06-2004 |
| | | | FR | 2766177 | A1 | 22-01-1999 |

EPO FORM P0460

Pour tout renseignement concernant cette annexe : voir Journal Officiel de l'Office européen des brevets, No.12/82

**ANNEXE AU RAPPORT DE RECHERCHE EUROPEENNE RELATIF A LA DEMANDE DE BREVET EUROPEEN NO.** EP 05 29 2741

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

17-03-2006

| Document brevet cité au rapport de recherche | | Date de publication | Membre(s) de la famille de brevet(s) | | | Date de publication |
|---|---|---|---|---|---|---|
| EP 0968171 | A | | WO | 9903819 | A1 | 28-01-1999 |
| | | | JP | 2000503036 | T | 14-03-2000 |
| | | | JP | 3379966 | B2 | 24-02-2003 |
| | | | JP | 2002053534 | A | 19-02-2002 |
| | | | JP | 2006022113 | A | 26-01-2006 |
| | | | US | 6565614 | B1 | 20-05-2003 |
| WO 9903836 | A | 28-01-1999 | AT | 277908 | T | 15-10-2004 |
| | | | AU | 8735598 | A | 10-02-1999 |
| | | | CA | 2265539 | A1 | 28-01-1999 |
| | | | DE | 69826624 | D1 | 04-11-2004 |
| | | | DE | 69826624 | T2 | 06-10-2005 |
| | | | EP | 0928289 | A1 | 14-07-1999 |
| | | | ES | 2230708 | T3 | 01-05-2005 |
| | | | FR | 2766178 | A1 | 22-01-1999 |
| | | | JP | 2000503037 | T | 14-03-2000 |
| | | | US | 6638321 | B1 | 28-10-2003 |
| EP 0928289 | A | 14-07-1999 | AT | 277908 | T | 15-10-2004 |
| | | | AU | 8735598 | A | 10-02-1999 |
| | | | CA | 2265539 | A1 | 28-01-1999 |
| | | | DE | 69826624 | D1 | 04-11-2004 |
| | | | DE | 69826624 | T2 | 06-10-2005 |
| | | | ES | 2230708 | T3 | 01-05-2005 |
| | | | FR | 2766178 | A1 | 22-01-1999 |
| | | | WO | 9903836 | A1 | 28-01-1999 |
| | | | JP | 2000503037 | T | 14-03-2000 |
| | | | US | 6638321 | B1 | 28-10-2003 |
| EP 1396486 | A | 10-03-2004 | FR | 2844271 | A1 | 12-03-2004 |
| WO 0197759 | A | 27-12-2001 | AT | 297180 | T | 15-06-2005 |
| | | | AU | 5226501 | A | 02-01-2002 |
| | | | CN | 1426295 | A | 25-06-2003 |
| | | | DE | 10020887 | A1 | 31-10-2001 |
| | | | EP | 1276451 | A1 | 22-01-2003 |
| | | | JP | 2003535879 | T | 02-12-2003 |
| | | | US | 2003150069 | A1 | 14-08-2003 |
| WO 9522312 | A | 24-08-1995 | AT | 201819 | T | 15-06-2001 |
| | | | DE | 4405127 | A1 | 31-08-1995 |
| | | | EP | 0744936 | A1 | 04-12-1996 |
| | | | JP | 9508910 | T | 09-09-1997 |
| | | | US | 6045779 | A | 04-04-2000 |

EPO FORM P0460

Pour tout renseignement concernant cette annexe : voir Journal Officiel de l'Office européen des brevets, No.12/82

## ANNEXE AU RAPPORT DE RECHERCHE EUROPEENNE RELATIF A LA DEMANDE DE BREVET EUROPEEN NO.

EP 05 29 2741

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

17-03-2006

| Document brevet cité au rapport de recherche | Date de publication | Membre(s) de la famille de brevet(s) | Date de publication |
|---|---|---|---|
| EP 1321131 A | 25-06-2003 | DE 10163803 A1 | 03-07-2003 |
| EP 1312346 A | 21-05-2003 | BR 0204962 A | 16-09-2003 |
| | | CA 2411096 A1 | 08-05-2003 |
| | | CN 1424013 A | 18-06-2003 |
| | | FR 2831810 A1 | 09-05-2003 |
| | | JP 2003160454 A | 03-06-2003 |
| | | MX PA02010943 A | 16-07-2004 |
| | | PL 357037 A1 | 19-05-2003 |
| | | US 2004010863 A1 | 22-01-2004 |
| | | ZA 200209096 A | 29-05-2003 |
| EP 1312345 A | 21-05-2003 | BR 0204978 A | 16-09-2003 |
| | | CA 2411446 A1 | 08-05-2003 |
| | | CN 1424014 A | 18-06-2003 |
| | | FR 2831812 A1 | 09-05-2003 |
| | | JP 2003146851 A | 21-05-2003 |
| | | MX PA02010940 A | 16-07-2004 |
| | | PL 357009 A1 | 19-05-2003 |
| | | US 2003121109 A1 | 03-07-2003 |
| | | ZA 200209051 A | 29-05-2003 |
| FR 2705888 A | 09-12-1994 | AU 6850794 A | 20-12-1994 |
| | | DE 69402976 D1 | 05-06-1997 |
| | | DE 69402976 T2 | 14-08-1997 |
| | | EP 0701433 A1 | 20-03-1996 |
| | | ES 2101582 T3 | 01-07-1997 |
| | | WO 9427572 A1 | 08-12-1994 |
| | | JP 8510464 T | 05-11-1996 |
| | | US 5756079 A | 26-05-1998 |

EPO FORM P0460

Pour tout renseignement concernant cette annexe : voir Journal Officiel de l'Office européen des brevets, No.12/82